# EUROPÄISCHE PATENTSCHRIFT

(11) **EP 3 335 253 B1**
(45) Veröffentlichungstag und Bekanntmachung des Hinweises auf die Patenterteilung: **21.08.2019**
(21) Anmeldenummer: 16739025.1
(22) Anmeldetag: 13.07.2016
(51) Int. Cl.: H01L 51/50, C09K 11/06, C07D 405/00, H01L 51/00, C07D 493/04, C09B 57/00, C09B 23/14, C09B 1/00, C07D 497/04

(54) **MATERIALIEN FÜR ELEKTRONISCHE VORRICHTUNGEN**
MATERIALS FOR ELECTRONIC DEVICES
MATÉRIAUX POUR DISPOSITIFS ÉLECTRONIQUES

(30) Priorität: 12.08.2015 EP 15180777
(43) Veröffentlichungstag der Anmeldung: 20.06.2018
(73) Patentinhaber: Merck Patent GmbH, 64293 Darmstadt (DE)
(72) Erfinder: RODRIGUEZ, Lara-Isabel, 64285 Darmstadt (DE); LINGE, Rouven, 64291 Darmstadt (DE); MEYER, Sebastian, 63741 Aschaffenburg (DE); HEIL, Holger, 60316 Frankfurt am Main (DE); BURKHART, Beate, 64293 Darmstadt (DE)
(86) Internationale Anmeldenummer: PCT/EP2016/001205
(87) Internationale Veröffentlichungsnummer: WO 2017/025165

(56) Entgegenhaltungen:
- WO-A1-2006/122630
- DE-A1-102006 025 846
- DE-A1-102009 052 428
- JP-A- 2010 045 281

## Beschreibung

Die vorliegende Anmeldung betrifft heteroaromatische Verbindungen einer unten näher definierten Formel (I), die sich zur Verwendung als Funktionsmaterialien in elektronischen Vorrichtungen eignen.

Unter dem Begriff elektronische Vorrichtung werden gemäß der vorliegenden Erfindung allgemein elektronische Vorrichtungen verstanden, welche organische Materialien enthalten. Bevorzugt werden darunter organische Elektrolumineszenzvorrichtungen (OLEDs) verstanden.

Betreffend die Leistungsdaten der elektronischen Vorrichtungen sind grundsätzlich weitere Verbesserungen erforderlich, insbesondere in Hinblick auf eine breite kommerzielle Verwendung, beispielsweise in Displays oder als Lichtquellen. Von besonderer Bedeutung sind in diesem Zusammenhang die Lebensdauer, die Effizienz und die Betriebsspannung der elektronischen Vorrichtungen sowie die realisierten Farbwerte. Insbesondere bei blau fluoreszierenden OLEDs besteht Verbesserungspotential bezüglich der Effizienz, der Lebensdauer der Vorrichtungen und den Farbwerten des emittierten Lichts.

Ein wichtiger Ansatzpunkt, um die genannten Verbesserungen zu erreichen, ist die Wahl der Verbindung, die als Matrix in der emittierenden Schicht der elektronischen Vorrichtung, bevorzugt in Kombination mit einer fluoreszierenden Emitterverbindung, eingesetzt wird. Dabei ist es von besonderem Interesse, dass die Verbindungen eine triplet-triplet annihilation (TTA) zulassen, da dadurch die Effizienz der Vorrichtung gesteigert wird.

Unter einer Matrix in der emittierenden Schicht werden im Rahmen der vorliegenden Anmeldung solche Verbindungen verstanden, die in der emittierenden Schicht der Vorrichtung vorhanden sind, jedoch keine Emitterverbindungen darstellen, d.h. nicht oder nur unwesentlich an der Lichtemission der emittierenden Schicht beteiligt sind.

Unter Emitterverbindungen werden entsprechend Verbindungen der emittierenden Schicht verstanden, welche beim Betrieb der Vorrichtung Licht emittieren. Vom Begriff fluoreszierende Emitter sind gemäß der vorliegenden Anmeldung Verbindungen umfasst, bei denen die Lichtemission aus einem Singulett-Zustand heraus erfolgt.

Im Rahmen der vorliegenden Erfindung wurde gefunden, dass sich Verbindungen der unten definierten Formel (I) hervorragend als Funktionsmaterialien für elektronische Vorrichtungen eignen, insbesondere eine hohe Quanteneffizienz, tiefblaue Farbkoordinaten, und eine lange Lebensdauer der Vorrichtungen bewirken. Weiterhin sind die Verbindungen hoch temperaturstabil. Nochmals weiterhin weisen die Verbindungen ein niedriges Triplet-Niveau auf, und eignen sich daher insbesondere für die Verwendung als Matrixverbindung in der emittierenden Schicht von OLEDs, in denen Triplet-Triplet-Annihilation stattfindet.

Gegenstand der Anmeldung ist daher eine Verbindung gemäß Formel (I) 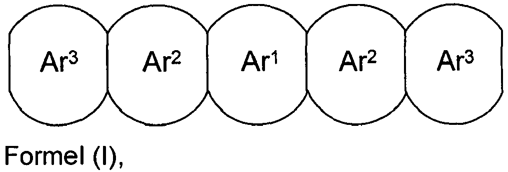 wobei für die auftretenden Variablen gilt: 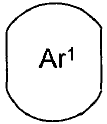 ist ein Benzolring, der jeweils mit Resten R¹ substituiert sein kann; 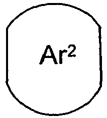 ist bei jedem Auftreten gleich oder verschieden gewählt aus Einheiten der Formel (Ar²-1) oder (Ar²-2) 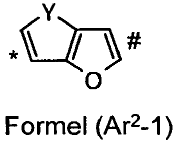 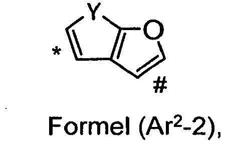 wobei die mit * markierte Bindung jeweils diejenige Bindung ist, über die die Einheit an die Gruppe Ar¹ ankondensiert ist, und die mit # markierte Bindung jeweils diejenige Bindung ist, über die die Einheit an die Gruppe Ar³ ankondensiert ist;
- Y: ist bei jedem Auftreten gleich oder verschieden C(R²)₂ oder Si(R²)₂; 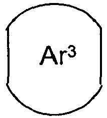 ist bei jedem Auftreten gleich oder verschieden gewählt aus aromatischen Ringsystemen mit 6 bis 30 aromatischen Ringatomen oder heteroaromatischen Ringsystemen mit 5 bis 30 aromatischen Ringatomen, die jeweils mit Resten R³ substituiert sein können;
- R¹, R², R³: sind bei jedem Auftreten gleich oder verschieden gewählt aus H, D, F, C(=O)R⁴, CN, Si(R⁴)₃, N(R⁴)₂, P(=O)(R⁴)₂, OR⁴, S(=O)R⁴, S(=O)₂R⁴, geradkettigen Alkyl- oder Alkoxygruppen mit 1 bis 20 C-Atomen, verzweigten oder cyclischen Alkyl- oder Alkoxygruppen mit 3 bis 20 C-Atomen, Alkenyl- oder Alkinylgruppen mit 2 bis 20 C-Atomen, aromatischen Ringsystemen mit 6 bis 40 aromatischen Ringatomen, und heteroaromatischen Ringsystemen mit 5 bis 40 aromatischen Ringatomen; wobei zwei oder mehr Reste R¹, R² bzw. R³ miteinander verknüpft sein können und einen Ring bilden können; wobei die genannten Alkyl-, Alkoxy-, Alkenyl- und Alkinylgruppen und die genannten aromatischen Ringsysteme und heteroaromatischen Ringsysteme jeweils mit einem oder mehreren Resten R⁴ substituiert sein können; und wobei eine oder mehrere CH₂-Gruppen in den genannten Alkyl-, Alkoxy-, Alkenyl- und Alkinylgruppen durch -R⁴C=CR⁴⁻, -C≡C-, Si(R⁴)₂, C=O, C=NR⁴, -C(=O)O-, -C(=O)NR⁴-, NR⁴, P(=O)(R⁴), -O-, -S-, SO oder SO₂ ersetzt sein können;
- R⁴: ist bei jedem Auftreten gleich oder verschieden gewählt aus H, D, F, C(=O)R⁵, CN, Si(R⁵)₃, N(R⁵)₂, P(=O)(R⁵)₂, OR⁵, S(=O)R⁵, S(=O)₂R⁵, geradkettigen Alkyl- oder Alkoxygruppen mit 1 bis 20 C-Atomen, verzweigten oder cyclischen Alkyl- oder Alkoxygruppen mit 3 bis 20 C-Atomen, Alkenyl- oder Alkinylgruppen mit 2 bis 20 C-Atomen, aromatischen Ringsystemen mit 6 bis 40 aromatischen Ringatomen, und heteroaromatischen Ringsystemen mit 5 bis 40 aromatischen Ringatomen; wobei zwei oder mehr Reste R⁴ miteinander verknüpft sein können und einen Ring bilden können; wobei die genannten Alkyl-, Alkoxy-, Alkenyl- und Alkinylgruppen und die genannten aromatischen Ringsysteme und heteroaromatischen Ringsysteme jeweils mit einem oder mehreren Resten R⁵ substituiert sein können; und wobei eine oder mehrere CH₂-Gruppen in den genannten Alkyl-, Alkoxy-, Alkenyl- und Alkinylgruppen durch -R⁵C=CR⁵-, -C≡C-, Si(R⁵)₂, C=O, C=NR⁵, -C(=O)O-, -C(=O)NR⁵-, NR⁵, P(=O)(R⁵), -O-, -S-, SO oder SO₂ ersetzt sein können;
- R⁵: ist bei jedem Auftreten gleich oder verschieden gewählt aus H, D, F, CN, Alkylgruppen mit 1 bis 20 C-Atomen, aromatischen Ringsystemen mit 6 bis 40 aromatischen Ringatomen und heteroaromatischen Ringsystemen mit 5 bis 40 aromatischen Ringatomen; wobei zwei oder mehr Reste R⁵ miteinander verknüpft sein können und einen Ring bilden können; und wobei die genannten Alkylgruppen, aromatischen Ringsysteme und heteroaromatischen Ringsysteme mit F oder CN substituiert sein können.

Im Folgenden werden die Gruppen 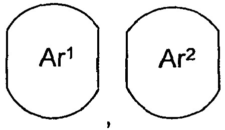 und 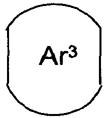 aus Gründen der Übersichtlichkeit ohne ihr grafisches Symbol, d.h. nur als "Ar¹", "Ar²", bzw. "Ar³" bezeichnet.

Zwei benachbarte Einheiten Ar¹, Ar² bzw. Ar³ sind dabei immer über eine gemeinsame Bindung miteinander kondensiert, in der Art, wie zwei Benzolringe über eine gemeinsame Bindung zu einer Naphthylgruppe kondensiert sind.

Bevorzugt sind die Bindungen, über die die Gruppen Ar¹, Ar² und Ar³ miteinander kondensiert sind, Bindungen zwischen zwei C-Atomen, bevorzugt zwischen zwei sp²-hybridisierten C-Atomen. Formel (I) entspricht damit folgender bevorzugter Formel (I-C)

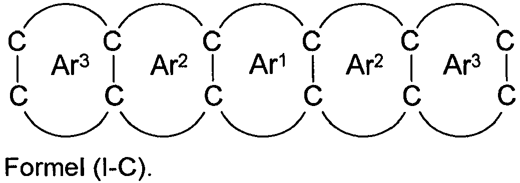

Eine Arylgruppe im Sinne dieser Erfindung enthält 6 bis 40 aromatische Ringatome, von denen keines ein Heteroatom darstellt. Unter einer Arylgruppe im Sinne dieser Erfindung wird entweder ein einfacher aromatischer Cyclus, also Benzol, oder ein kondensierter aromatischer Polycyclus, beispielsweise Naphthalin, Phenanthren oder Anthracen, verstanden. Ein kondensierter aromatischer Polycyclus besteht im Sinne der vorliegenden Anmeldung aus zwei oder mehr miteinander kondensierten einfachen aromatischen Cyclen. Unter Kondensation zwischen Cyclen ist dabei zu verstehen, dass die Cyclen mindestens eine Kante miteinander teilen.

Eine Heteroarylgruppe im Sinne dieser Erfindung enthält 5 bis 40 aromatische Ringatome, von denen mindestens eines ein Heteroatom darstellt. Die Heteroatome der Heteroarylgruppe sind bevorzugt ausgewählt aus N, O und S. Unter einer Heteroarylgruppe im Sinne dieser Erfindung wird entweder ein einfacher heteroaromatischer Cyclus, beispielsweise Pyridin, Pyrimidin oder Thiophen, oder ein kondensierter heteroaromatischer Polycyclus, beispielsweise Chinolin oder Carbazol, verstanden. Ein kondensierter heteroaromatischer Polycyclus besteht im Sinne der vorliegenden Anmeldung aus zwei oder mehr miteinander kondensierten einfachen heteroaromatischen Cyclen. Unter Kondensation zwischen Cyclen ist dabei zu verstehen, dass die Cyclen mindestens eine Kante miteinander teilen.

Unter einer Aryl- oder Heteroarylgruppe, die jeweils mit den oben genannten Resten substituiert sein kann und die über beliebige Positionen am Aromaten bzw. Heteroaromaten verknüpft sein kann, werden insbesondere Gruppen verstanden, welche abgeleitet sind von Benzol, Naphthalin, Anthracen, Phenanthren, Pyren, Dihydropyren, Chrysen, Perylen, Triphenylen, Fluoranthen, Benzanthracen, Benzphenanthren, Tetracen, Pentacen, Benzpyren, Furan, Benzofuran, Isobenzofuran, Dibenzofuran, Thiophen, Benzothiophen, Isobenzothiophen, Dibenzothiophen, Pyrrol, Indol, Isoindol, Carbazol, Pyridin, Chinolin, Isochinolin, Acridin, Phenanthridin, Benzo-5,6-chinolin, Benzo-6,7-chinolin, Benzo-7,8-chinolin, Phenothiazin, Phenoxazin, Pyrazol, Indazol, Imidazol, Benzimidazol, Naphthimidazol, Phenanthrimidazol, Pyridimidazol, Pyrazinimidazol, Chinoxalinimidazol, Oxazol, Benzoxazol, Naphthoxazol, Anthroxazol, Phenanthroxazol, Isoxazol, 1,2-Thiazol, 1,3-Thiazol, Benzothiazol, Pyridazin, Benzopyridazin, Pyrimidin, Benzpyrimidin, Chinoxalin, Pyrazin, Phenazin, Naphthyridin, Azacarbazol, Benzocarbolin, Phenanthrolin, 1,2,3-Triazol, 1,2,4-Triazol, Benzotriazol, 1,2,3-Oxadiazol, 1,2,4-Oxadiazol, 1,2,5-Oxadiazol, 1,3,4-Oxadiazol, 1,2,3-Thiadiazol, 1,2,4-Thiadiazol, 1,2,5-Thiadiazol, 1,3,4-Thiadiazol, 1,3,5-Triazin, 1,2,4-Triazin, 1,2,3-Triazin, Tetrazol, 1,2,4,5-Tetrazin, 1,2,3,4-Tetrazin, 1,2,3,5-Tetrazin, Purin, Pteridin, Indolizin und Benzothiadiazol.

Ein aromatisches Ringsystem im Sinne dieser Erfindung enthält 6 bis 40 C-Atome im Ringsystem und umfasst keine Heteroatome als aromatische Ringatome. Ein aromatisches Ringsystem im Sinne dieser Erfindung enthält daher keine Heteroarylgruppen. Unter einem aromatischen Ringsystem im Sinne dieser Erfindung soll ein System verstanden werden, das nicht notwendigerweise nur Arylgruppen enthält, sondern in dem auch mehrere Arylgruppen durch eine Einfachbindung oder durch eine nicht-aromatische Einheit, wie beispielsweise ein oder mehrere wahlweise substituierte C-, Si-, N-, O- oder S-Atome, verbunden sein können. Dabei umfasst die nicht-aromatische Einheit bevorzugt weniger als 10 % der von H verschiedenen Atome, bezogen auf die Gesamtzahl der von H verschiedenen Atome des Systems. So sollen beispielsweise auch Systeme wie 9,9'-Spirobifluoren, 9,9'-Diarylfluoren, Triarylamin, Diarylether und Stilben als aromatische Ringsysteme im Sinne dieser Erfindung verstanden werden, und ebenso Systeme, in denen zwei oder mehr Arylgruppen beispielsweise durch eine lineare oder cyclische Alkyl-, Alkenyl- oder Alkinylgruppe oder durch eine Silylgruppe verbunden sind. Weiterhin werden auch Systeme, in denen zwei oder mehr Arylgruppen über Einfachbindungen miteinander verknüpft sind, als aromatische Ringsysteme im Sinne dieser Erfindung verstanden, wie beispielsweise Systeme wie Biphenyl und Terphenyl.

Ein heteroaromatisches Ringsystem im Sinne dieser Erfindung enthält 5 bis 40 aromatische Ringatome, von denen mindestens eines ein Heteroatom darstellt. Die Heteroatome des heteroaromatischen Ringsystems sind bevorzugt ausgewählt aus N, O und/oder S. Ein heteroaromatisches Ringsystem entspricht der oben genannten Definition eines aromatischen Ringsystems, weist jedoch mindestens ein Heteroatom als eines der aromatischen Ringatome auf. Es unterscheidet sich dadurch von einem aromatischen Ringsystem im Sinne der Definition der vorliegenden Anmeldung, welches gemäß dieser Definition kein Heteroatom als aromatisches Ringatom enthalten kann.

Unter einem aromatischen Ringsystem mit 6 bis 40 aromatischen Ringatomen oder einem heteroaromatischen Ringsystem mit 5 bis 40 aromatischen Ringatomen, werden insbesondere Gruppen verstanden, die abgeleitet sind von den oben unter Arylgruppen und Heteroarylgruppen genannten Gruppen sowie von Biphenyl, Terphenyl, Quaterphenyl, Fluoren, Spirobifluoren, Dihydrophenanthren, Dihydropyren, Tetrahydropyren, Indenofluoren, Truxen, Isotruxen, Spirotruxen, Spiroisotruxen, Indenocarbazol, oder von Kombinationen dieser Gruppen.

Im Rahmen der vorliegenden Erfindung werden unter einer geradkettigen Alkylgruppe mit 1 bis 20 C-Atomen bzw. einer verzweigten oder cyclischen Alkylgruppe mit 3 bis 20 C-Atomen bzw. einer Alkenyl- oder Alkinylgruppe mit 2 bis 40 C-Atomen, in der auch einzelne H-Atome oder CH₂-Gruppen durch die oben bei der Definition der Reste genannten Gruppen substituiert sein können, bevorzugt die Reste Methyl, Ethyl, n-Propyl, i-Propyl, n-Butyl, i-Butyl, s-Butyl, t-Butyl, 2-Methylbutyl, n-Pentyl, s-Pentyl, Cyclopentyl, neo-Pentyl, n-Hexyl, Cyclohexyl, neo-Hexyl, n-Heptyl, Cycloheptyl, n-Octyl, Cyclooctyl, 2-Ethylhexyl, Trifluormethyl, Pentafluorethyl, 2,2,2-Trifluorethyl, Ethenyl, Propenyl, Butenyl, Pentenyl, Cyclopentenyl, Hexenyl, Cyclohexenyl, Heptenyl, Cycloheptenyl, Octenyl, Cyclooctenyl, Ethinyl, Propinyl, Butinyl, Pentinyl, Hexinyl oder Octinyl verstanden.

Unter einer Alkoxy- oder Thioalkylgruppe mit 1 bis 20 C-Atomen, in der auch einzelne H-Atome oder CH₂-Gruppen durch die oben bei der Definition der Reste genannten Gruppen substituiert sein können, werden bevorzugt Methoxy, Trifluormethoxy, Ethoxy, n-Propoxy, i-Propoxy, n-Butoxy, i-Butoxy, s-Butoxy, t-Butoxy, n-Pentoxy, s-Pentoxy, 2-Methylbutoxy, n-Hexoxy, Cyclohexyloxy, n-Heptoxy, Cycloheptyloxy, n-Octyloxy, Cyclooctyloxy, 2-Ethylhexyloxy, Pentafluorethoxy, 2,2,2-Trifluorethoxy, Methylthio, Ethylthio, n-Propylthio, i-Propylthio, n-Butylthio, i-Butylthio, s-Butylthio, t-Butylthio, n-Pentylthio, s-Pentylthio, n-Hexylthio, Cyclohexylthio, n-Heptylthio, Cycloheptylthio, n-Octylthio, Cyclooctylthio, 2-Ethylhexylthio, Trifluormethylthio, Pentafluorethylthio, 2,2,2-Trifluorethylthio, Ethenylthio, Propenylthio, Butenylthio, Pentenylthio, Cyclopentenylthio, Hexenylthio, Cyclohexenylthio, Heptenylthio, Cycloheptenylthio, Octenylthio, Cyclooctenylthio, Ethinylthio, Propinylthio, Butinylthio, Pentinylthio, Hexinylthio, Heptinylthio oder Octinylthio verstanden.

Unter der Formulierung, dass zwei oder mehr Reste miteinander einen Ring bilden können, soll im Rahmen der vorliegenden Anmeldung unter anderem verstanden werden, dass die beiden Reste miteinander durch eine chemische Bindung verknüpft sind. Weiterhin soll unter der oben genannten Formulierung aber auch verstanden werden, dass für den Fall, dass einer der beiden Reste Wasserstoff darstellt, der zweite Rest unter Bildung eines Rings an die Position, an die das Wasserstoffatom gebunden war, bindet.

Bevorzugt sind die Verbindungen der Formel (I) symmetrisch, bezogen auf eine Spiegelebene durch die Mitte und senkrechtstehend auf der Längsachse des langgestreckten Moleküls, insbesondere durch die Mitte der Gruppe Ar¹. Dies bedeutet, dass die rechte Seite und die linke Seite des Moleküls, gesehen jeweils von der Mitte, d.h. von der Gruppe Ar¹ aus, gleich sind. Besonders bevorzugt gilt dies nicht nur für das Grundgerüst, sondern auch für die gesamte Verbindung, d.h. unter Einbeziehung der Substituenten. Es sind jedoch von der allgemeinen Formel auch asymmetrische Verbindungen umfasst, insbesondere solche, die bezogen auf das Grundgerüst der Formel (I) symmetrisch sind, aufgrund ihrer Substitution jedoch im Ganzen asymmetrisch sind.

Bevorzugt entspricht Ar¹ einer der unten gezeigten Formeln (Ar¹-1) und (Ar¹-2) 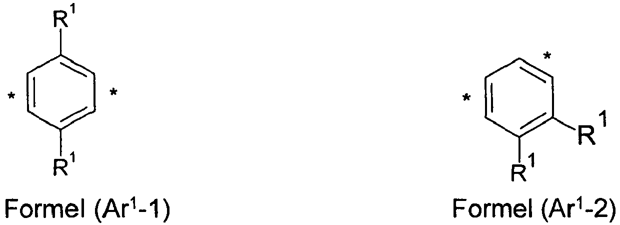 wobei die mit * markierten Bindungen diejenigen Bindungen sind, über die die betreffende Gruppe Ar¹ an die beiden benachbarten Gruppen Ar² ankondensiert ist.

Besonders bevorzugt ist unter den Formeln (Ar¹-1) und (Ar¹-2) die Formel (Ar¹-1).

Bevorzugt sind in Formel (Ar¹-1) und (Ar¹-2) die Gruppen R¹ gleich H.

Bevorzugt ist Ar² gewählt aus Gruppen der Formel (Ar²-1).

Bevorzugt ist die Gruppe Y gleich C(R²)₂. Besonders bevorzugt ist die Wahl von Ar² entsprechend Formel (Ar²-1), und die gleichzeitige Wahl von Y als C(R²)₂.

Bevorzugt ist Ar³ bei jedem Auftreten gleich oder verschieden gewählt aus Benzol, Pyridin, Pyrimidin, Pyrazin, Pyridazin, Pyrrol, Furan, Thiophen, Benzofuran, Benzothiophen, Dibenzofuran, Dibenzothiophen, Fluoren, Spirobifluoren, Indenofluoren, Naphthalin und Anthracen, die jeweils mit Resten R³ substituiert sein können. Besonders bevorzugt sind darunter Benzol, Naphthalin, Fluoren und Spirobifluoren, die jeweils mit Resten R³ substituiert sein können.

Bevorzugte Gruppen Ar³ sind gewählt aus den folgenden Gruppen: 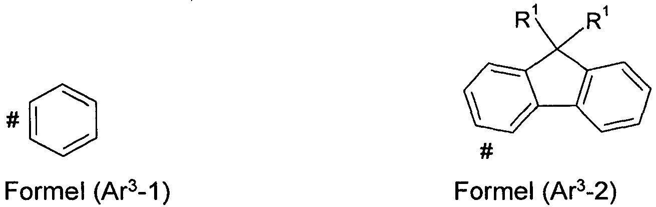 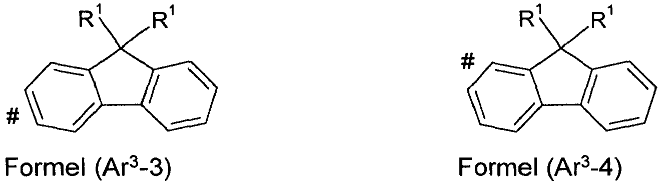 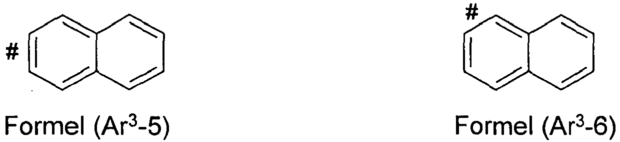 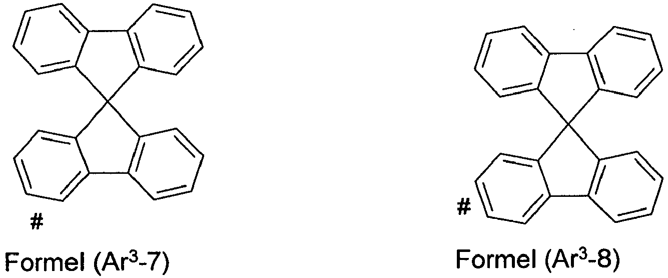 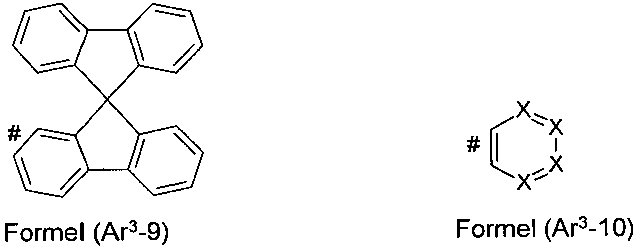 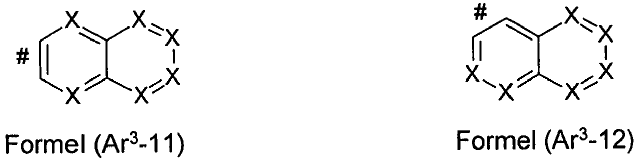 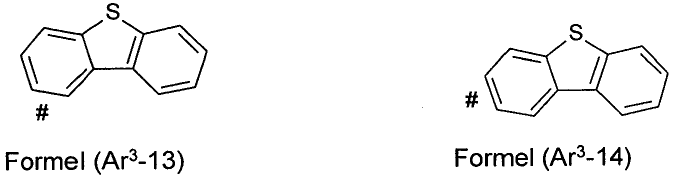 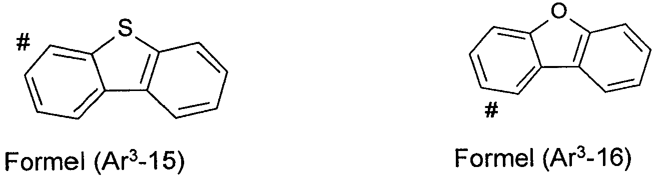 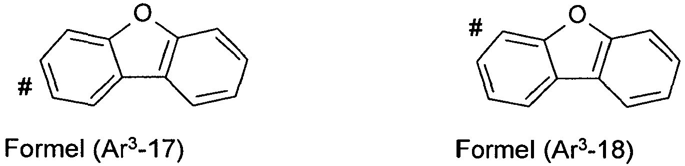 wobei die mit # markierte Bindung diejenige Bindung ist, über die die betreffende Gruppe Ar¹ an die benachbarte Gruppe Ar² ankondensiert ist, wobei die Gruppen an den unsubstituiert gezeigten Positionen mit Resten R³ substituiert sein können, und wobei X bei jedem Auftreten gleich oder verschieden N oder CR³ ist.

Bevorzugt sind nicht mehr als drei Gruppen X in einem Sechsring gleich N. Weiterhin bevorzugt sind nicht mehr als zwei benachbarte Gruppen C gleich N.

Bevorzugte Gruppen R¹ sind bei jedem Auftreten gleich oder verschieden gewählt aus H, F, CN, Alkylgruppen mit 1 bis 20 C-Atomen, aromatischen Ringsystemen mit 6 bis 30 aromatischen Ringatomen und heteroaromatischen Ringsystemen mit 5 bis 30 aromatischen Ringatomen; wobei zwei oder mehr Reste R¹ miteinander verknüpft sein können und einen Ring bilden können; und wobei die genannten Alkylgruppen, aromatischen Ringsysteme und heteroaromatischen Ringsysteme mit R⁴ substituiert sein können und bevorzugt mit F oder CN substituiert sein können.

Bevorzugte Gruppen R² sind bei jedem Auftreten gleich oder verschieden gewählt aus geradkettigen Alkylgruppen mit 1 bis 20 C-Atomen, verzweigten oder cyclischen Alkylgruppen mit 3 bis 20 C-Atomen, aromatischen Ringsystemen mit 6 bis 18 aromatischen Ringatomen, und heteroaromatischen Ringsystemen mit 5 bis 18 aromatischen Ringatomen, wobei die genannten Gruppen mit Resten R⁴ substituiert sein können. Besonders bevorzugte Gruppen R² sind bei jedem Auftreten gleich oder verschieden gewählt aus Methyl, Ethyl, Propyl, Butyl, Pentyl, Hexyl, Heptyl, Octyl, Nonyl, Decyl, und Phenyl, die jeweils mit Resten R⁴ substituiert sein können und bevorzugt unsubstituiert sind.

Bevorzugte Gruppen R³ sind bei jedem Auftreten gleich oder verschieden gewählt aus H, F, CN, Alkylgruppen mit 1 bis 20 C-Atomen, aromatischen Ringsystemen mit 6 bis 30 aromatischen Ringatomen und heteroaromatischen Ringsystemen mit 5 bis 30 aromatischen Ringatomen; wobei zwei oder mehr Reste R³ miteinander verknüpft sein können und einen Ring bilden können; und wobei die genannten Alkylgruppen, aromatischen Ringsysteme und heteroaromatischen Ringsysteme mit R⁴ substituiert sein können und bevorzugt mit F oder CN substituiert sein können.

Bevorzugte Ausführungsformen der Formel (I) entsprechen der folgenden Formel 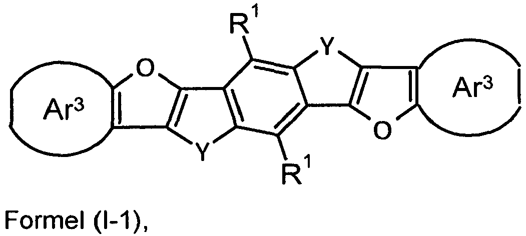 wobei die auftretenden Symbole definiert sind wie obenstehend, und wobei die Gruppe Y bevorzugt gleich C(R²)₂ ist, und wobei die Gruppen Ar³ bevorzugt den oben angegebenen Gruppen der Formeln (Ar³-1) bis (Ar³-9) entsprechen.

Die Gruppe Ar³ ist in der bevorzugten Formel (I-1), wie obenstehend für Formel (I) erläutert, über eine gemeinsame Bindung mit der Furan-Einheit kondensiert.

Bevorzugt gelten für Formel (I-1) die oben angegebenen bevorzugten Ausführungsformen der variablen Gruppen.

Als Ausführungsform der anmeldungsgemäßen Verbindungen ist besonders bevorzugt eine Ausführungsform, für die die folgenden kombinierten Bedingungen gelten:
- Das Grundgerüst entspricht der Formel (I-1);
- Y ist gleich C(R²)₂;
- Die Gruppen Ar³ entsprechen einer Formel ausgewählt aus den Formeln (Ar³-1) bis (Ar³-9), bevorzugt der Formel (Ar³-1).

Die Verbindung der Formel (I) ist bevorzugt dadurch gekennzeichnet, dass der Wert für ihr Tripletniveau größer ist als der durch 2 geteilte Wert für ihr Singulett-Niveau. Die Werte für Singulett-Energieniveau und Triplet-Energieniveau werden dabei durch quantenmechanische Rechnung bestimmt, wie in den Ausführungsbeispielen der WO 2015/036080, Abschnitt A), angegeben.

Folgende Verbindungen sind Beispiele für Verbindungen gemäß Formel (I):

| | |
|---|---|
| 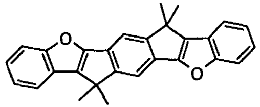 | 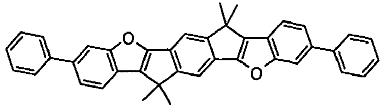 |
| (1) | (2) |
| 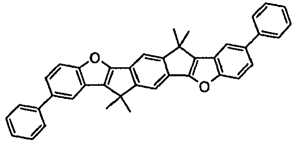 | 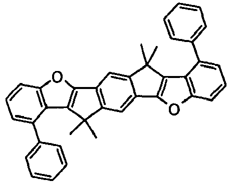 |
| (3) | (4) |
| 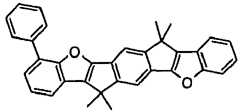 | 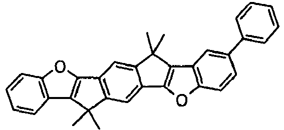 |
| (5) | (6) |
| 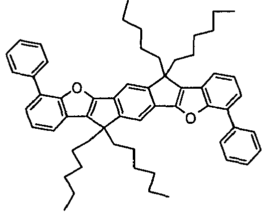 | 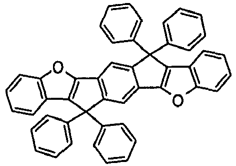 |
| (7) | (8) |

| | |
|---|---|
| 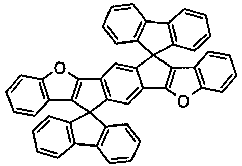 | 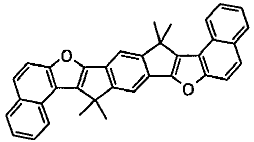 |
| (9) | (10) |
| 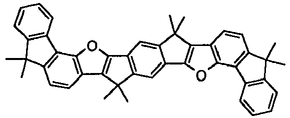 | 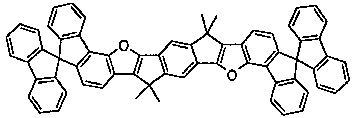 |
| (11) | (12) |
| 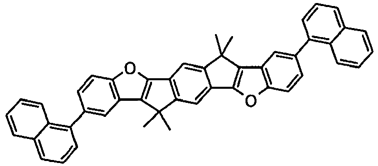 | 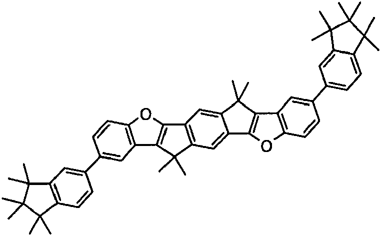 |
| (13) | (14) |
| 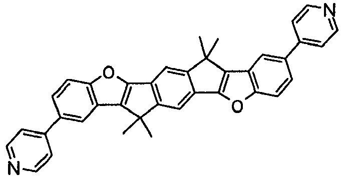 | 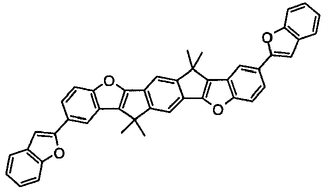 |
| (15) | (16) |
| 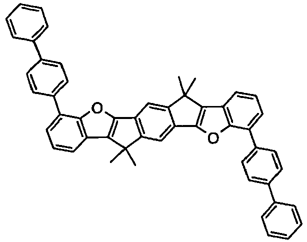 | 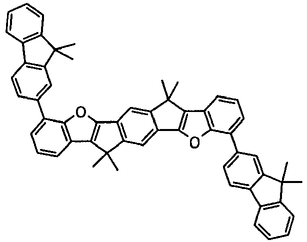 |
| (17) | (18) |

| | |
|---|---|
| 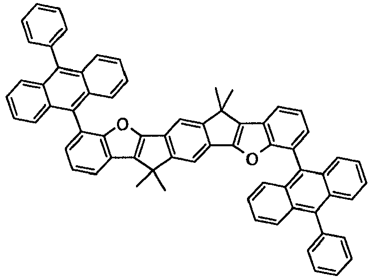 | 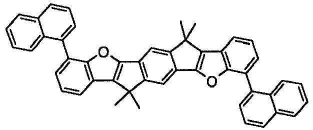 |
| (19) | (20) |
| 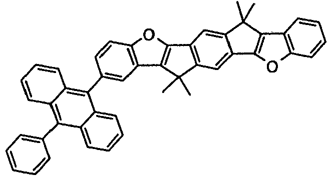 | 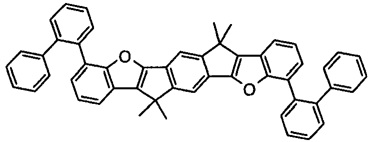 |
| (21) | (22) |
| 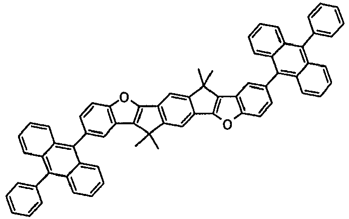 | 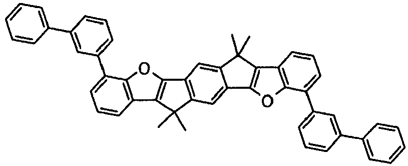 |
| (23) | (24) |
| 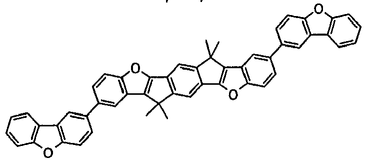 | 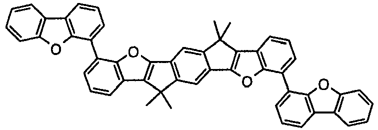 |
| (25) | (26) |
| 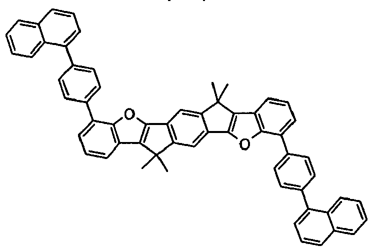 | |
| (27) | |

Die Verbindungen gemäß der vorliegenden Anmeldung können beispielsweise wie folgt hergestellt werden (vgl. allgemeines Schema der Ausführungsbeispiele unter A-1)):
Hierzu werden zunächst in einem ersten Schritt zwei Furan-enthaltende Gruppen I, die auch die Einheit Ar³ umfassen, mit der zentralen Einheit Ar¹ über eine metallorganische Kupplungsreaktion verbunden. Die zentrale Einheit ist dabei difunktionell, so dass zwei Äquivalente der Furanenthaltenden Verbindung mit einem Äquivalent der zentralen Einheit Ar¹ reagieren.

In einem zweiten Schritt wird die doppelt auftretende Ringschlussreaktion, die die Einheiten Ar² bildet, vorbereitet. Dies geschieht durch Reduktion zweier Estergruppen an der Einheit Ar¹ zu einer tertiären Alkoholgruppe, bevorzugt durch eine Alkylmagnesiumverbindung.

In einem dritten Schritt wird die doppelt auftretende Ringschlussreaktion vollzogen, durch Hinzufügen von Säure. Dadurch wird das Grundgerüst der Verbindung der Formel (I) erhalten. Dieses kann, wie im allgemeinen Schema unter A-2 gezeigt, weiter modifiziert werden, bevorzugt durch Bromierung und anschließende Einführung von aromatischen Gruppen durch metallorganische Kupplungsreaktion.

Die Synthese von Verbindungen der Formel (I), welche Silyl-Brücken enthalten (Y = Si(R²)₂), kann beispielsweise gemäß den in Q.-W. Zhang et al., Synlett 2015, 26, 1145-1152 beschriebenen Verfahren erfolgen.

Für die Synthese von unsymmetrisch substituierten Verbindungen (vgl. untenstehendes Schema) kann von einem mit zwei unterschiedlichen Halogengruppen substituierten Terephthalsäurederivat ausgegangen werden, welches sequenziell zunächst mit einer Benzofuran-Einheit, und dann mit einer zweiten Benzofuraneinheit verbunden wird. Die weiteren Schritte (Reduktion zum tertiären Alkohol und Ringschluss unter Säureeinwirkung) entsprechen denjenigen, die in dem Schema in A-1) für die symmetrischen Derivate gezeigt sind. Auf diese Weise können Verbindungen erhalten werden, welche unterschiedliche Substituenten an den beiden endständigen Benzofurangruppen tragen.

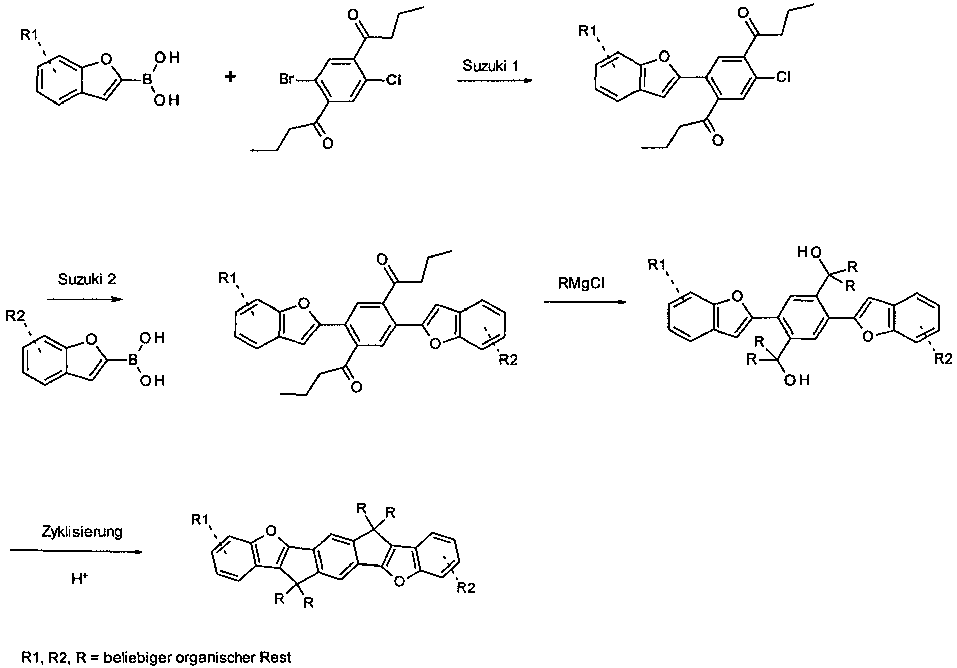

Gegenstand der vorliegenden Anmeldung ist daher auch ein Verfahren zur Herstellung von Verbindungen der Formel (I), dadurch gekennzeichnet, dass die Verbindung durch die folgenden, in der vorliegenden Reihenfolge durchgeführten Schritte gebildet wird:
i) eine metallorganische Kupplungsreaktion zwischen zwei Verbindungen, die je eine Furangruppe enthalten, und einer Verbindung enthaltend die Einheit Ar¹,
ii) die anschließende Reduktion einer in der Verbindung vorhandenen, an die Einheit Ar¹ gebundenen Estergruppe zu einer an ebendiese Einheit Ar¹ gebundenen tertiären Alkoholgruppe, und schließlich
iii) eine Ringschlussreaktion dieser tertiären Alkoholgruppe unter Bildung einer Alkylenbrücke zwischen der Einheit Ar¹ und dem Furanring.

Die oben beschriebenen erfindungsgemäßen Verbindungen, insbesondere Verbindungen, welche mit reaktiven Abgangsgruppen, wie Brom, Iod, Chlor, Boronsäure oder Boronsäureester, substituiert sind, können als Monomere zur Erzeugung entsprechender Oligomere, Dendrimere oder Polymere Verwendung finden. Geeignete reaktive Abgangsgruppen sind beispielsweise Brom, Iod, Chlor, Boronsäuren, Boronsäureester, Amine, Alkenyl- oder Alkinylgruppen mit endständiger C-C-Doppelbindung bzw. C-C-Dreifachbindung, Oxirane, Oxetane, Gruppen, die eine Cycloaddition, beispielsweise eine 1,3-dipolare Cycloaddition, eingehen, wie beispielsweise Diene oder Azide, Carbonsäurederivate, Alkohole und Silane.

Weiterer Gegenstand der Erfindung sind daher Oligomere, Polymere oder Dendrimere enthaltend eine oder mehrere Verbindungen gemäß Formel (I), wobei die Bindung(en) zum Polymer, Oligomer oder Dendrimer an beliebigen, in Formel (I) mit R¹, R² oder R³ substituierten Positionen lokalisiert sein können. Je nach Verknüpfung der Verbindung gemäß Formel (I) ist die Verbindung Bestandteil einer Seitenkette des Oligomers oder Polymers oder Bestandteil der Hauptkette. Unter einem Oligomer im Sinne dieser Erfindung wird eine Verbindung verstanden, welche aus mindestens drei Monomereinheiten aufgebaut ist. Unter einem Polymer im Sinne der Erfindung wird eine Verbindung verstanden, die aus mindestens zehn Monomereinheiten aufgebaut ist. Die erfindungsgemäßen Polymere, Oligomere oder Dendrimere können konjugiert, teilkonjugiert oder nichtkonjugiert sein. Die erfindungsgemäßen Oligomere oder Polymere können linear, verzweigt oder dendritisch sein. In den linear verknüpften Strukturen können die Einheiten gemäß Formel (I) direkt miteinander verknüpft sein oder sie können über eine bivalente Gruppe, beispielsweise über eine substituierte oder unsubstituierte Alkylengruppe, über ein Heteroatom oder über eine bivalente aromatische oder heteroaromatische Gruppe miteinander verknüpft sein. In verzweigten und dendritischen Strukturen können beispielsweise drei oder mehrere Einheiten gemäß Formel (I) über eine trivalente oder höhervalente Gruppe, beispielsweise über eine trivalente oder höhervalente aromatische oder heteroaromatische Gruppe, zu einem verzweigten bzw. dendritischen Oligomer oder Polymer verknüpft sein.

Die entsprechenden Polymere enthaltend Einheiten der Formel (I) können beispielsweise als Triplet-Controll-Polymer für Triplet-Triplet-Annihilation verwendet werden. Die Gruppen der Formel (I) können dabei Anthracen-Gruppen gleichwertig ersetzen.

Für die Wiederholeinheiten gemäß Formel (I) in Oligomeren, Dendrimeren und Polymeren gelten dieselben Bevorzugungen wie oben für Verbindungen gemäß Formel (I) beschrieben.

Zur Herstellung der Oligomere oder Polymere werden die erfindungsgemäßen Monomere homopolymerisiert oder mit weiteren Monomeren copolymerisiert. Geeignete und bevorzugte Comonomere sind gewählt aus Fluorenen (z. B. gemäß EP 842208 oder WO 00/22026), Spirobifluorenen (z. B. gemäß EP 707020, EP 894107 oder WO 06/061181), Paraphenylenen (z. B. gemäß WO 1992/18552), Carbazolen (z. B. gemäß WO 04/070772 oder WO 2004/113468), Thiophenen (z. B. gemäß EP 1028136), Dihydrophenanthrenen (z. B. gemäß WO 2005/014689 oder WO 2007/006383), cis- und trans-Indenofluorenen (z. B. gemäß WO 2004/041901 oder WO 2004/113412), Ketonen (z. B. gemäß WO 2005/040302), Phenanthrenen (z. B. gemäß WO 2005/104264 oder WO 2007/017066) oder auch mehreren dieser Einheiten. Die Polymere, Oligomere und Dendrimere enthalten üblicherweise noch weitere Einheiten, beispielsweise emittierende (fluoreszierende oder phosphoreszierende) Einheiten, wie z. B. Vinyltriarylamine (z. B. gemäß WO 2007/068325) oder phosphoreszierende Metallkomplexe (z. B. gemäß WO 2006/003000), und/oder Ladungstransporteinheiten, insbesondere solche basierend auf Triarylaminen.

Die erfindungsgemäßen Polymere und Oligomere werden in der Regel durch Polymerisation von einer oder mehreren Monomersorten hergestellt, von denen mindestens ein Monomer im Polymer zu Wiederholungseinheiten der Formel (I) führt. Geeignete Polymerisationsreaktionen sind dem Fachmann bekannt und in der Literatur beschrieben. Besonders geeignete und bevorzugte Polymerisationsreaktionen, die zu C-C- bzw. C-N-Verknüpfungen führen, sind folgende:
(A) SUZUKI-Polymerisation;
(B) YAMAMOTO-Polymerisation;
(C) STILLE-Polymerisation; und
(D) HARTWIG-BUCHWALD-Polymerisation.

Wie die Polymerisation nach diesen Methoden durchgeführt werden kann und wie die Polymere dann vom Reaktionsmedium abgetrennt und aufgereinigt werden können, ist dem Fachmann bekannt und in der Literatur, beispielsweise in WO 2003/048225, WO 2004/037887 und WO 2004/037887, im Detail beschrieben.

Für die Verarbeitung der erfindungsgemäßen Verbindungen aus flüssiger Phase, beispielsweise durch Spin-Coating oder durch Druckverfahren, sind Formulierungen der erfindungsgemäßen Verbindungen erforderlich. Diese Formulierungen können beispielsweise Lösungen, Dispersionen oder Emulsionen sein. Es kann bevorzugt sein, hierfür Mischungen aus zwei oder mehr Lösemitteln zu verwenden. Geeignete und bevorzugte Lösemittel sind beispielsweise Toluol, Anisol, o-, m- oder p-Xylol, Methylbenzoat, Mesitylen, Tetralin, Veratrol, THF, Methyl-THF, THP, Chlorbenzol, Dioxan, Phenoxytoluol, insbesondere 3-Phenoxytoluol, (-)-Fenchon, 1,2,3,5-Tetramethylbenzol, 1,2,4,5-Tetramethylbenzol, 1-Methylnaphthalin, 2-Methylbenzothiazol, 2-Phenoxyethanol, 2-Pyrrolidinon, 3-Methylanisol, 4-Methylanisol, 3,4-Dimethylanisol, 3,5-Dimethylanisol, Acetophenon, α-Terpineol, Benzothiazol, Butylbenzoat, Cumol, Cyclohexanol, Cyclohexanon, Cyclohexylbenzol, Decalin, Dodecylbenzol, Ethylbenzoat, Indan, Methylbenzoat, NMP, p-Cymol, Phenetol, 1,4-Diisopropylbenzol, Dibenzylether, Diethylenglycolbutylmethylether, Triethylenglycolbutylmethyl-ether, Diethylenglycoldibutylether, Triethylenglycoldimethylether, Diethylenglycolmonobutylether, Tripropylenglycoldimethylether, Tetraethylenglycoldimethylether, 2-Isopropylnaphthalin, Pentylbenzol, Hexylbenzol, Heptylbenzol, Octylbenzol, 1,1-Bis(3,4-Dimethylphenyl)ethan oder Mischungen dieser Lösemittel.

Gegenstand der Erfindung ist daher weiterhin eine Formulierung, insbesondere eine Lösung, Dispersion oder Emulsion, enthaltend mindestens eine Verbindung gemäß Formel (I) oder mindestens ein Polymer, Oligomer oder Dendrimer enthaltend mindestens eine Einheit gemäß Formel (I) sowie mindestens ein Lösungsmittel, bevorzugt ein organisches Lösungsmittel. Wie solche Lösungen hergestellt werden können, ist dem Fachmann bekannt und beispielsweise in WO 2002/072714, WO 2003/019694 und der darin zitierten Literatur beschrieben.

Die erfindungsgemäßen Verbindungen eignen sich für den Einsatz in elektronischen Vorrichtungen, insbesondere in organischen Elektrolumineszenzvorrichtungen (OLEDs). Abhängig von der Substitution werden die Verbindungen in unterschiedlichen Funktionen und Schichten eingesetzt.

Die erfindungsgemäßen Verbindungen können in jeder Funktion in der organischen Elektrolumineszenzvorrichtung eingesetzt werden, beispielsweise als Matrixmaterial, als emittierendes Material, als lochtransportierendes Material oder als elektronentransportierendes Material. Bevorzugt ist die Verwendung als Matrixmaterial in einer emittierenden Schicht, bevorzugt einer fluoreszierenden emittierenden Schicht, die Verwendung als Lochtransportmaterial in einer lochtransportierenden Schicht einer OLED, und die Verwendung als emittierendes Material, bevorzugt als blau fluoreszierendes Material, in einer emittierenden Schicht.

Weiterer Gegenstand der Erfindung ist daher die Verwendung einer Verbindung gemäß Formel (I) in einer elektronischen Vorrichtung. Dabei ist die elektronische Vorrichtung bevorzugt ausgewählt aus der Gruppe bestehend aus organischen integrierten Schaltungen (OICs), organischen Feld-Effekt-Transistoren (OFETs), organischen Dünnfilmtransistoren (OTFTs), organischen lichtemittierenden Transistoren (OLETs), organischen Solarzellen (OSCs), organischen optischen Detektoren, organischen Photorezeptoren, organischen Feld-Quench-Devices (OFQDs), organischen lichtemittierenden elektrochemischen Zellen (OLECs), organischen Laserdioden (O-Laser) und besonders bevorzugt organischen Elektrolumineszenzvorrichtungen (OLEDs).

Weiterer Gegenstand der Erfindung ist eine elektronische Vorrichtung, enthaltend mindestens eine Verbindung der Formel (I). Bevorzugt ist die elektronische Vorrichtung gewählt aus den oben angegebenen Vorrichtungen. Besonders bevorzugt ist eine organische Elektrolumineszenzvorrichtung, enthaltend Anode, Kathode und mindestens eine emittierende Schicht, dadurch gekennzeichnet, dass mindestens eine organische Schicht mindestens eine Verbindung gemäß Formel (I) enthält. Ganz besonders bevorzugt ist eine organische Elektrolumineszenzvorrichtung, enthaltend Anode, Kathode und mindestens eine emittierende Schicht, enthaltend mindestens eine Verbindung gemäß Formel (I).

Außer Kathode, Anode und der emittierenden Schicht kann die organische Elektrolumineszenzvorrichtung noch weitere Schichten enthalten. Diese sind beispielsweise gewählt aus jeweils einer oder mehreren Lochinjektionsschichten, Lochtransportschichten, Lochblockierschichten, Elektronentransportschichten, Elektroneninjektionsschichten, Elektronenblockierschichten, Excitonenblockierschichten, Zwischenschichten (Interlayers), Ladungserzeugungsschichten (Charge-Generation Layers) (IDMC 2003, Taiwan; Session 21 OLED (5), T. Matsumoto, T. Nakada, J. Endo, K. Mori, N. Kawamura, A. Yokoi, J. Kido, Multiphoton Organic EL Device Having Charge Generation Layer) und/oder organischen oder anorganischen p/n-Übergängen.

Die Abfolge der Schichten der organischen Elektrolumineszenzvorrichtung ist bevorzugt die folgende: Anode-Lochinjektionsschicht-Lochtransportschicht-emittierende Schicht-Elektronentransportschicht-Elektroneninjektionsschicht-Kathode. Dabei müssen nicht alle der genannten Schichten vorhanden sein, und es können zusätzlich weitere Schichten vorhanden sein, beispielsweise eine Elektronenblockierschicht anodenseitig an die emittierende Schicht angrenzend, oder eine Lochblockierschicht kathodenseitig an die emittierende Schicht angrenzend.

Die erfindungsgemäße organische Elektrolumineszenzvorrichtung kann mehrere emittierende Schichten enthalten. Besonders bevorzugt weisen diese Emissionsschichten in diesem Fall insgesamt mehrere Emissionsmaxima zwischen 380 nm und 750 nm auf, so dass insgesamt weiße Emission resultiert, d. h. in den emittierenden Schichten werden verschiedene emittierende Verbindungen verwendet, die fluoreszieren oder phosphoreszieren können und die blaues oder grünes oder gelbes oder orangefarbenes oder rotes Licht emittieren. Insbesondere bevorzugt sind Dreischichtsysteme, also Systeme mit drei emittierenden Schichten, wobei bevorzugt mindestens eine dieser Schichten mindestens eine Verbindung gemäß Formel (I) enthält und wobei die drei Schichten blaue, grüne und orange oder rote Emission zeigen (für den prinzipiellen Aufbau siehe z. B. WO 2005/011013). Es soll angemerkt werden, dass sich für die Erzeugung von weißem Licht anstelle mehrerer farbig emittierender Emitterverbindungen auch eine einzeln verwendete Emitterverbindung eignen kann, welche in einem breiten Wellenlängenbereich emittiert. Alternativ und/oder zusätzlich können in einer derartigen organischen Elektrolumineszenzvorrichtung die erfindungsgemäßen Verbindungen auch in der Lochtransportschicht oder in einer anderen Schicht vorhanden sein.

Die erfindungsgemäße Verbindung eignet sich insbesondere zur Verwendung als Matrixverbindung für eine Emitterverbindung, bevorzugt eine blau emittierende Emitterverbindung, besonders bevorzugt für eine blau fluoreszierende Emitterverbindung.

Wird die Verbindung als Matrixverbindung in einer emittierenden Schicht verwendet, ist es bevorzugt, dass sie Triplet-Triplet-Annihilation bzw. Triplet-Triplet-Fusion erlaubt. Darunter versteht man einen Mechanismus, bei dem je zwei Tripletzustände zu einem Singulett-Zustand kombinieren, wodurch sich die Effizienz der Singulett-OLED erhöht. Bevorzugt ist daher, wenn die Verbindung der Formel (I) als Matrixmaterial in der emittierenden Schicht eingesetzt wird, dass die Triplett-Niveaus der Materialien der an die emittierende Schicht angrenzenden Schichten größer sind als die der Verbindung der Formel (I). Weiterhin ist es bevorzugt, dass das Triplet-Niveau der emittierenden Verbindung, die zusammen mit der Verbindung der Formel (I) in der emittierenden Schicht vorliegt, höher liegt als das Triplet-Niveau der Verbindung der Formel (I).

Wird die erfindungsgemäße Verbindung als Matrixmaterial eingesetzt, kann sie in Kombination mit beliebigen, dem Fachmann bekannten emittierenden Verbindungen eingesetzt werden. Bevorzugt wird sie in Kombination mit den unten angegebenen bevorzugten emittierenden Verbindungen eingesetzt, besonders den unten angegebenen bevorzugten fluoreszierenden Verbindungen.

Für den Fall, dass die emittierende Schicht der organischen Elektrolumineszenzvorrichtung eine Mischung aus einer emittierenden Verbindung und einer Matrixverbindung enthält, gilt folgendes: Der Anteil der emittierenden Verbindung in der Mischung der emittierenden Schicht beträgt bevorzugt zwischen 0.1 und 50.0 %, besonders bevorzugt zwischen 0.5 und 20.0 %, und ganz besonders bevorzugt zwischen 1.0 und 10.0 %. Entsprechend beträgt der Anteil des Matrixmaterials bzw. der Matrixmaterialien bevorzugt zwischen 50.0 und 99.9 %, besonders bevorzugt zwischen 80.0 und 99.5 %, und ganz besonders bevorzugt zwischen 90.0 und 99.0 %.

Dabei wird unter den Angaben der Anteile in % im Rahmen der vorliegenden Anmeldung Vol.-% verstanden, wenn die Verbindungen aus der Gasphase aufgebracht werden, und es wird darunter Gew.-% verstanden, wenn die Verbindungen aus Lösung aufgebracht werden.

Gemäß einer weiteren bevorzugten Ausführungsform der Erfindung werden die Verbindungen der Formel (I) als Lochtransportmaterialien in einer lochtransportierenden Schicht eingesetzt. Dies kann eine beliebige zwischen Anode und emittierender Schicht angeordnete Schicht sein, beispielsweise eine Lochinjektionsschicht, eine Lochtransportschicht oder eine Elektronenblockierschicht. Bevorzugt ist es eine Lochtransportschicht, d.h. eine Schicht, die sich zwischen der Lochinjektionsschicht und der Elektronenblockierschicht bzw. der emittierenden Schicht befindet.

Wird die Verbindung gemäß Formel (I) als Lochtransportmaterial in einer Lochtransportschicht, einer Lochinjektionsschicht oder einer Elektronenblockierschicht verwendet, so kann die Verbindung als Reinmaterial, d.h. in einem Anteil von 100 %, in der Lochtransportschicht eingesetzt werden, oder sie kann in Kombination mit einer oder mehreren weiteren Verbindungen eingesetzt werden. Gemäß einer bevorzugten Ausführungsform enthält die organische Schicht enthaltend die Verbindung der Formel (I) dann zusätzlich einen oder mehrere p-Dotanden. Als p-Dotanden werden gemäß der vorliegenden Erfindung bevorzugt solche organischen Elektronenakzeptorverbindungen eingesetzt, die eine oder mehrere der anderen Verbindungen der Mischung oxidieren können.

Wird die Verbindung der Formel (I) als emittierende Verbindung in einer emittierenden Schicht eingesetzt, so ist sie bevorzugt eine blau fluoreszierende emittierende Verbindung. Bevorzugt wird sie in diesem Fall in Kombination mit mindestens einer weiteren Verbindung (Matrixverbindung) in der emittierenden Schicht verwendet. Dabei gelten für die Anteile der emittierenden Verbindung der Formel (I) und der Matrixverbindung die oben angegebenen bevorzugten Ausführungsformen. Bevorzugte Matrixverbindungen zur Kombination mit der Verbindung der Formel (I) in ihrer Funktion als emittierende Verbindung sind gewählt aus den im Folgenden genannten bevorzugten Klassen von Matrixverbindungen.

Im Folgenden sind allgemein bevorzugte Materialklassen zur Verwendung als entsprechende Funktionsmaterialien in den erfindungsgemäßen organischen Elektrolumineszenzvorrichtungen aufgeführt.

Als phosphoreszierende emittierende Verbindungen eignen sich insbesondere Verbindungen, die bei geeigneter Anregung Licht, vorzugsweise im sichtbaren Bereich, emittieren und außerdem mindestens ein Atom der Ordnungszahl größer 20, bevorzugt größer 38 und kleiner 84, besonders bevorzugt größer 56 und kleiner 80 enthalten. Bevorzugt werden als phosphoreszierende emittierende Verbindungen Verbindungen, die Kupfer, Molybdän, Wolfram, Rhenium, Ruthenium, Osmium, Rhodium, Iridium, Palladium, Platin, Silber, Gold oder Europium enthalten, verwendet, insbesondere Verbindungen, die Iridium, Platin oder Kupfer enthalten.

Dabei werden im Sinne der vorliegenden Erfindung alle lumineszierenden Iridium-, Platin- oder Kupferkomplexe als phosphoreszierende Verbindungen angesehen.

Beispiele der oben beschriebenen phosphoreszierenden emittierenden Verbindungen können den Anmeldungen WO 2000/70655, WO 2001/41512, WO 2002/02714, WO 2002/15645, EP 1191613, EP 1191612, EP 1191614, WO 2005/033244, WO 2005/019373 und US 2005/0258742 entnommen werden. Generell eignen sich alle phosphoreszierenden Komplexe, wie sie gemäß dem Stand der Technik für phosphoreszierende OLEDs verwendet werden und wie sie dem Fachmann auf dem Gebiet der organischen Elektrolumineszenzvorrichtungen bekannt sind, zur Verwendung in den erfindungsgemäßen Vorrichtungen. Auch kann der Fachmann ohne erfinderisches Zutun weitere phosphoreszierende Komplexe in Kombination mit den erfindungsgemäßen Verbindungen in OLEDs einsetzen.

Bevorzugte fluoreszierende Emitter sind neben den erfindungsgemäßen Verbindungen ausgewählt aus der Klasse der Arylamine. Unter einem Arylamin im Sinne dieser Erfindung wird eine Verbindung verstanden, die drei substituierte oder unsubstituierte aromatische oder heteroaromatische Ringsysteme direkt an den Stickstoff gebunden enthält. Bevorzugt ist mindestens eines dieser aromatischen oder heteroaromatischen Ringsysteme ein kondensiertes Ringsystem, besonders bevorzugt mit mindestens 14 aromatischen Ringatomen. Bevorzugte Beispiele hierfür sind aromatische Anthracenamine, aromatische Anthracendiamine, aromatische Pyrenamine, aromatische Pyrendiamine, aromatische Chrysenamine oder aromatische Chrysendiamine. Unter einem aromatischen Anthracenamin wird eine Verbindung verstanden, in der eine Diarylaminogruppe direkt an eine Anthracengruppe gebunden ist, vorzugsweise in 9-Position. Unter einem aromatischen Anthracendiamin wird eine Verbindung verstanden, in der zwei Diarylaminogruppen direkt an eine Anthracengruppe gebunden sind, vorzugsweise in 9,10-Position. Aromatische Pyrenamine, Pyrendiamine, Chrysenamine und Chrysendiamine sind analog dazu definiert, wobei die Diarylaminogruppen am Pyren bevorzugt in 1-Position bzw. in 1,6-Position gebunden sind. Weitere bevorzugte Emitter sind Indenofluorenamine bzw. -diamine, beispielsweise gemäß WO 2006/108497 oder WO 2006/122630, Benzoindenofluorenamine bzw. -diamine, beispielsweise gemäß WO 2008/006449, und Dibenzoindenofluorenamine bzw. -diamine, beispielsweise gemäß WO 2007/140847, sowie die in WO 2010/012328 offenbarten Indenofluorenderivate mit kondensierten Arylgruppen. Ebenfalls bevorzugt sind die in WO 2012/048780 und WO 2013/185871 offenbarten Pyren-Arylamine. Ebenfalls bevorzugt sind die in WO 2014/037077 offenbarten Benzoindenofluoren-Amine, die in WO 2014/106522 offenbarten Benzofluoren-Amine und die in WO 2014/111269 offenbarten erweiterten Indenofluorene.

Bevorzugte fluoreszierende emittierende Verbindungen sind in der folgenden Tabelle abgebildet:

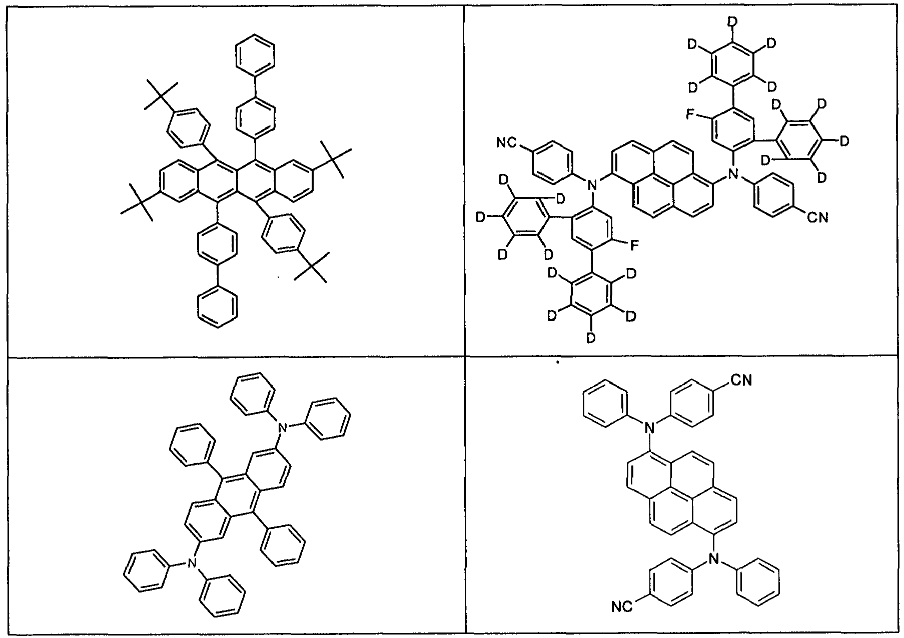

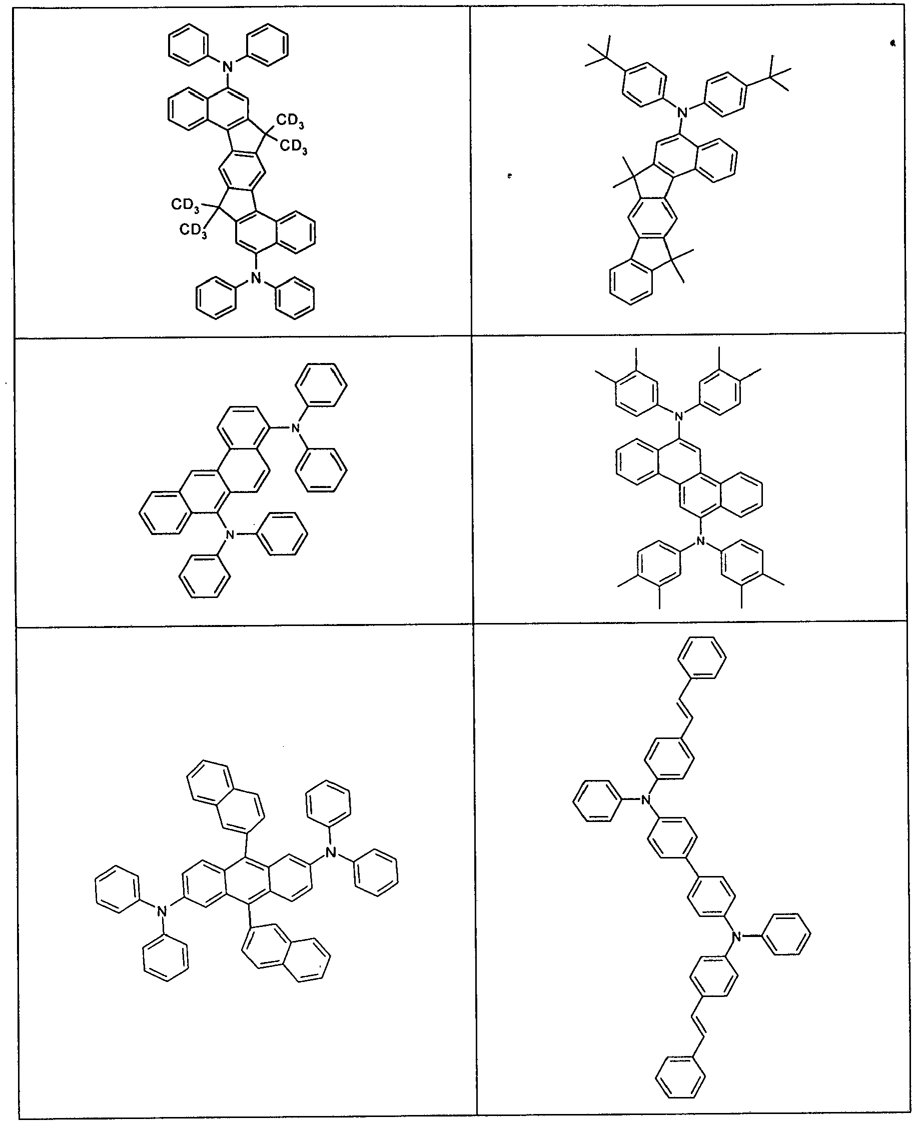

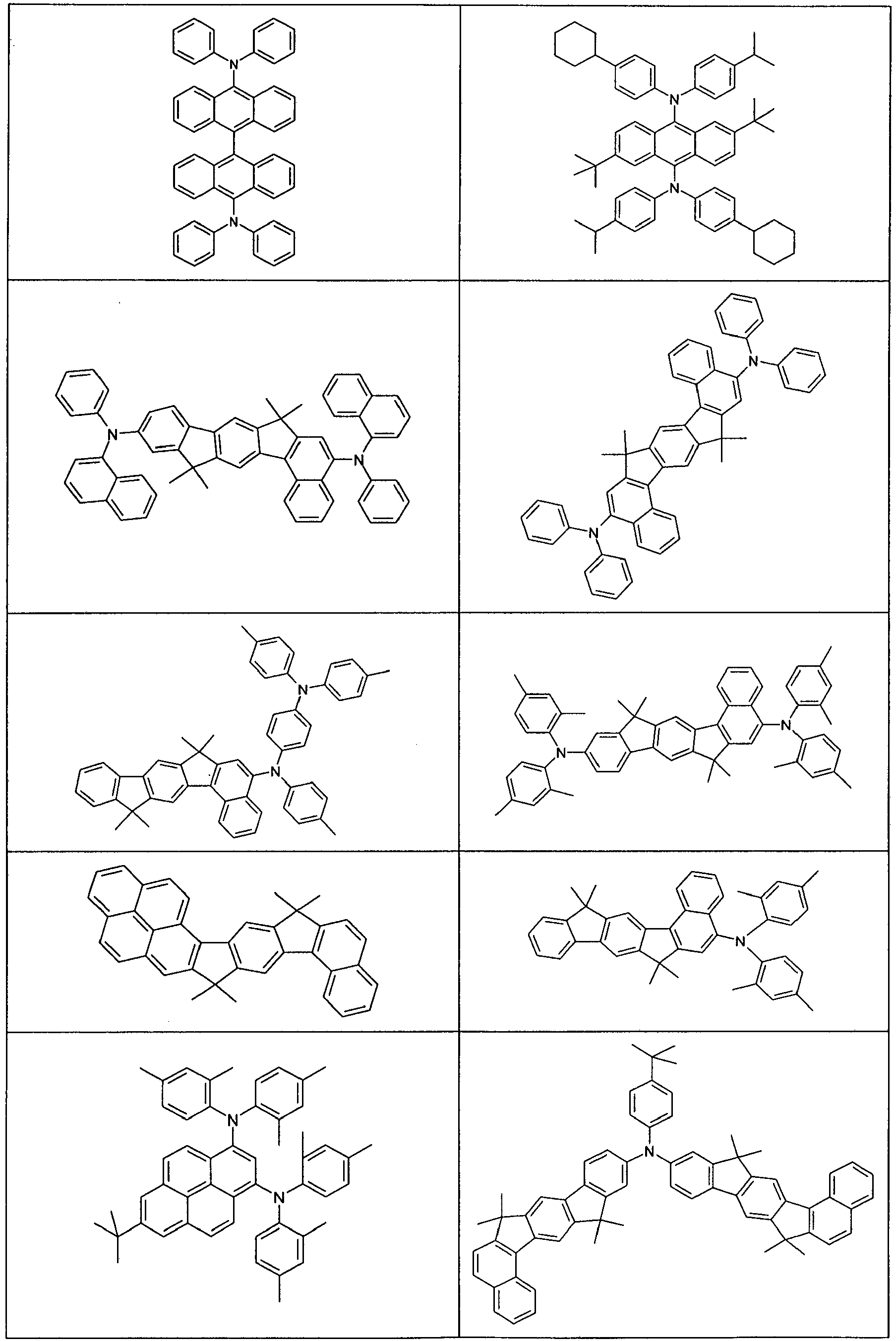

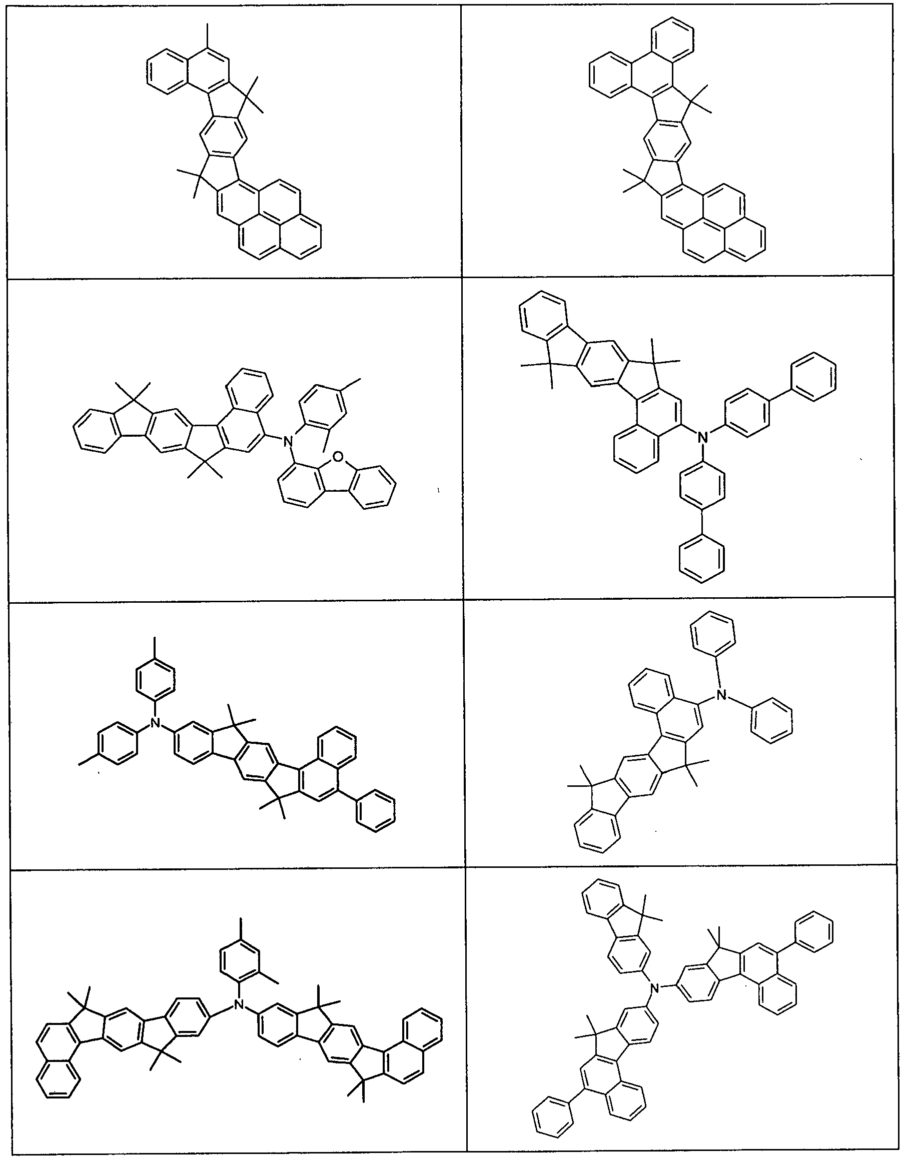

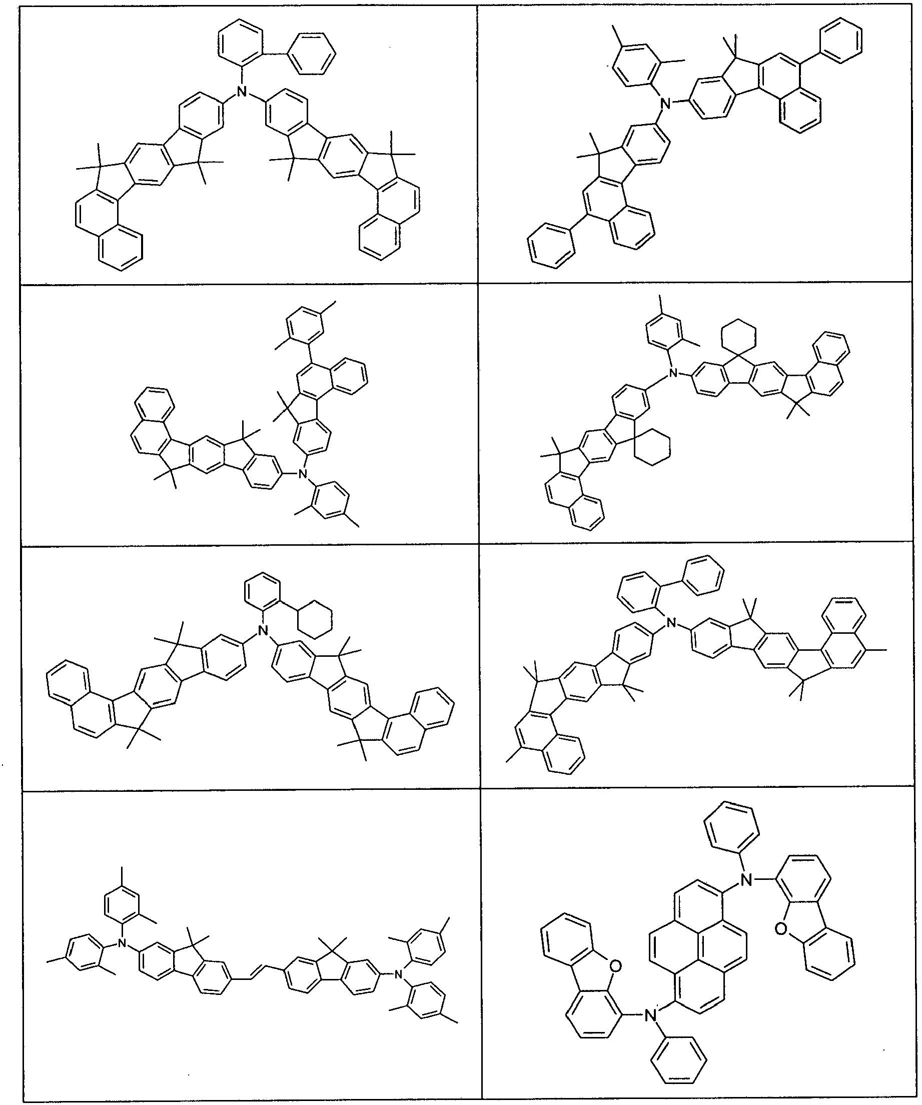

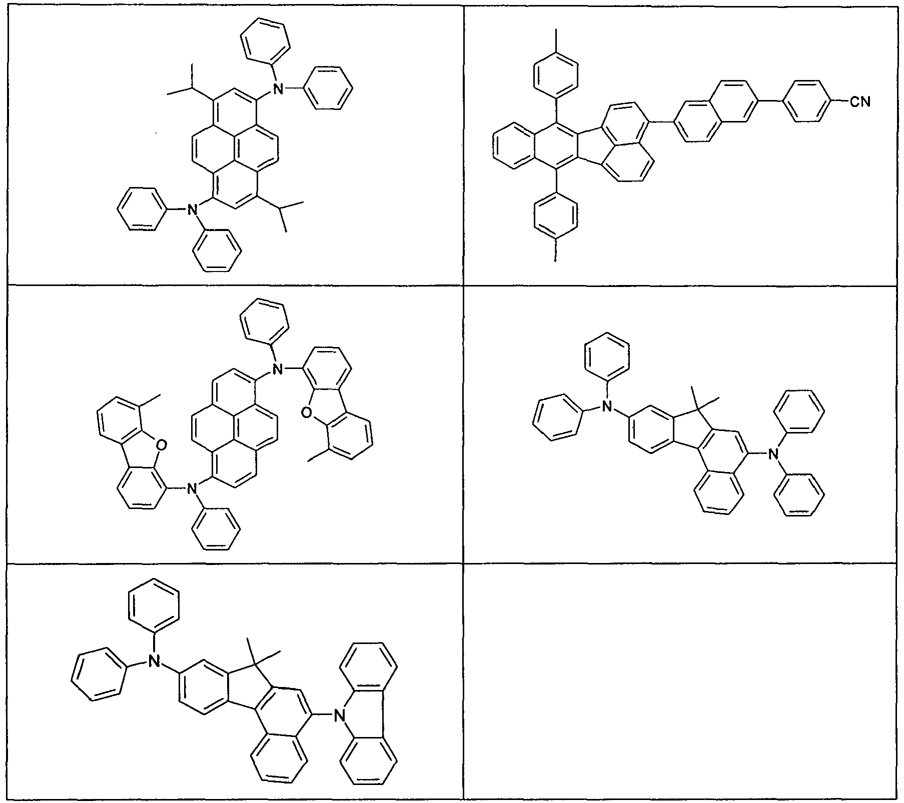

Bevorzugte Matrixmaterialien für phosphoreszierende emittierende Verbindungen sind aromatische Amine, insbesondere Triarylamine, z. B. gemäß US 2005/0069729, Carbazolderivate (z. B. CBP, N,N-Biscarbazolylbiphenyl) oder Verbindungen gemäß WO 2005/039246, US 2005/0069729, JP 2004/288381, EP 1205527 oder WO 2008/086851, verbrückte Carbazolderivate, z. B. gemäß WO 2011/088877 und WO 2011/128017, Indenocarbazolderivate, z. B. gemäß WO 2010/136109 und WO 2011/000455, Azacarbazolderivate, z. B. gemäß EP 1617710, EP 1617711, EP 1731584, JP 2005/347160, Indolocarbazolderivate, z. B. gemäß WO 2007/063754 oder WO 2008/056746, Ketone, z. B. gemäß WO 2004/093207 oder WO 2010/006680, Phosphinoxide, Sulfoxide und Sulfone, z. B. gemäß WO 2005/003253, Oligophenylene, bipolare Matrixmaterialien, z. B. gemäß WO 2007/137725, Silane, z. B. gemäß WO 2005/111172, Azaborole oder Boronester, z. B. gemäß WO 2006/117052, Triazinderivate, z. B. gemäß WO 2010/015306, WO 2007/063754 oder WO 2008/056746, Zinkkomplexe, z. B. gemäß EP 652273 oder WO 2009/062578, Aluminiumkomplexe, z. B. BAlq, Diazasilol- und Tetraazasilol-Derivate, z. B. gemäß WO 2010/054729 und Diazaphosphol-Derivate, z. B. gemäß WO 2010/054730.

Bevorzugte Matrixmaterialien zur Verwendung in Kombination mit fluoreszierenden emittierenden Verbindungen sind neben den Verbindungen der Formel (I) ausgewählt aus den Klassen der Oligoarylene (z. B. 2,2',7,7'-Tetraphenylspirobifluoren gemäß EP 676461 oder Dinaphthylanthracen), insbesondere der Oligoarylene enthaltend kondensierte aromatische Gruppen, der Oligoarylenvinylene (z. B. DPVBi oder Spiro-DPVBi gemäß EP 676461), der polypodalen Metallkomplexe (z. B. gemäß WO 2004/081017), der lochleitenden Verbindungen (z. B. gemäß WO 2004/058911), der elektronenleitenden Verbindungen, insbesondere Ketone, Phosphinoxide, Sulfoxide, etc. (z. B. gemäß WO 2005/084081 und WO 2005/084082), der Atropisomere (z. B. gemäß WO 2006/048268), der Boronsäurederivate (z. B. gemäß WO 2006/117052) oder der Benzanthracene (z. B. gemäß WO 2008/145239). Besonders bevorzugte Matrixmaterialien sind ausgewählt aus den Klassen der Oligoarylene, enthaltend Naphthalin, Anthracen, Benzanthracen und/oder Pyren oder Atropisomere dieser Verbindungen, der Oligoarylenvinylene, der Ketone, der Phosphinoxide und der Sulfoxide. Ganz besonders bevorzugte Matrixmaterialien sind ausgewählt aus den Klassen der Oligoarylene, enthaltend Anthracen, Benzanthracen, Benzphenanthren und/oder Pyren oder Atropisomere dieser Verbindungen. Unter einem Oligoarylen im Sinne dieser Erfindung soll eine Verbindung verstanden werden, in der mindestens drei Aryl- bzw. Arylengruppen aneinander gebunden sind.

Geeignete Ladungstransportmaterialien, wie sie in der Lochinjektions-bzw. Lochtransportschicht bzw. Elektronenblockierschicht oder in der Elektronentransportschicht der erfindungsgemäßen organischen Elektrolumineszenzvorrichtung verwendet werden können, sind neben den erfindungsgemäßen Verbindungen beispielsweise die in Y. Shirota et al., Chem. Rev. 2007, 107(4), 953-1010 offenbarten Verbindungen oder andere Materialien, wie sie gemäß dem Stand der Technik in diesen Schichten eingesetzt werden.

Beispiele für bevorzugte Lochtransportmaterialien, die in einer Lochtransport-, Lochinjektions- oder Elektronenblockierschicht in der erfindungsgemäßen Elektrolumineszenzvorrichtung verwendet werden können, sind neben der Verbindungen der Formel (I) Indenofluorenamin-Derivate (z. B. gemäß WO 06/122630 oder WO 06/100896), die in EP 1661888 offenbarten Aminderivate, Hexaazatriphenylenderivate (z. B. gemäß WO 01/049806), Aminderivate mit kondensierten Aromaten (z. B. gemäß US 5,061,569), die in WO 95/09147 offenbarten Aminderivate, Monobenzoindenofluorenamine (z. B. gemäß WO 08/006449), Dibenzoindenofluorenamine (z. B. gemäß WO 07/140847), Spirobifluoren-Amine (z. B. gemäß WO 2012/034627 oder WO 2013/120577), Fluoren-Amine (z. B. gemäß WO 2014/015937, WO 2014/015938 und WO 2014/015935), Spiro-Dibenzopyran-Amine (z. B. gemäß WO 2013/083216) und Dihydroacridin-Derivate (z. B. gemäß WO 2012/150001).

Als Kathode der organischen Elektrolumineszenzvorrichtung sind Metalle mit geringer Austrittsarbeit, Metalllegierungen oder mehrlagige Strukturen aus verschiedenen Metallen bevorzugt, wie beispielsweise Erdalkalimetalle, Alkalimetalle, Hauptgruppenmetalle oder Lanthanoide (z. B. Ca, Ba, Mg, AI, In, Mg, Yb, Sm, etc.). Weiterhin eignen sich Legierungen aus einem Alkali- oder Erdalkalimetall und Silber, beispielsweise eine Legierung aus Magnesium und Silber. Bei mehrlagigen Strukturen können auch zusätzlich zu den genannten Metallen weitere Metalle verwendet werden, die eine relativ hohe Austrittsarbeit aufweisen, wie z. B. Ag oder Al, wobei dann in der Regel Kombinationen der Metalle, wie beispielsweise Ca/Ag, Mg/Ag oder Ba/Ag verwendet werden. Es kann auch bevorzugt sein, zwischen einer metallischen Kathode und dem organischen Halbleiter eine dünne Zwischenschicht eines Materials mit einer hohen Dielektrizitätskonstante einzubringen. Hierfür kommen beispielsweise Alkalimetall- oder Erdalkalimetallfluoride, aber auch die entsprechenden Oxide oder Carbonate in Frage (z. B. LiF, Li₂O, BaF₂, MgO, NaF, CsF, Cs₂CO₃, etc.). Weiterhin kann dafür Lithiumchinolinat (LiQ) verwendet werden. Die Schichtdicke dieser Schicht beträgt bevorzugt zwischen 0.5 und 5 nm.

Als Anode sind Materialien mit hoher Austrittsarbeit bevorzugt. Bevorzugt weist die Anode eine Austrittsarbeit größer 4.5 eV vs. Vakuum auf. Hierfür sind einerseits Metalle mit hohem Redoxpotential geeignet, wie beispielsweise Ag, Pt oder Au. Es können andererseits auch Metall/Metalloxid-Elektroden (z. B. Al/Ni/NiOₓ, Al/PtOₓ) bevorzugt sein. Für einige Anwendungen muss mindestens eine der Elektroden transparent oder teiltransparent sein, um entweder die Bestrahlung des organischen Materials (organische Solarzelle) oder die Auskopplung von Licht (OLED, O-LASER) zu ermöglichen. Bevorzugte Anodenmaterialien sind hier leitfähige gemischte Metalloxide. Besonders bevorzugt sind Indium-ZinnOxid (ITO) oder Indium-Zink Oxid (IZO). Bevorzugt sind weiterhin leitfähige, dotierte organische Materialien, insbesondere leitfähige dotierte Polymere.

Die Vorrichtung wird entsprechend (je nach Anwendung) strukturiert, kontaktiert und schließlich versiegelt, da sich die Lebensdauer der erfindungsgemäßen Vorrichtungen bei Anwesenheit von Wasser und/oder Luft verkürzt.

In einer bevorzugten Ausführungsform ist die erfindungsgemäße organische Elektrolumineszenzvorrichtung dadurch gekennzeichnet, dass eine oder mehrere Schichten mit einem Sublimationsverfahren beschichtet werden. Dabei werden die Materialien in Vakuum-Sublimationsanlagen bei einem Anfangsdruck kleiner 10⁻⁵ mbar, bevorzugt kleiner 10⁻⁶ mbar aufgedampft. Dabei ist es jedoch auch möglich, dass der Anfangsdruck noch geringer ist, beispielsweise kleiner 10⁻⁷ mbar.

Bevorzugt ist ebenfalls eine organische Elektrolumineszenzvorrichtung, dadurch gekennzeichnet, dass eine oder mehrere Schichten mit dem OVPD (Organic Vapour Phase Deposition) Verfahren oder mit Hilfe einer Trägergassublimation beschichtet werden. Dabei werden die Materialien bei einem Druck zwischen 10⁻⁵ mbar und 1 bar aufgebracht. Ein Spezialfall dieses Verfahrens ist das OVJP (Organic Vapour Jet Printing) Verfahren, bei dem die Materialien direkt durch eine Düse aufgebracht und so strukturiert werden (z. B. M. S. Arnold et al., Appl. Phys. Lett. 2008, 92, 053301).

Weiterhin bevorzugt ist eine organische Elektrolumineszenzvorrichtung, dadurch gekennzeichnet, dass eine oder mehrere Schichten aus Lösung, wie z. B. durch Spincoating, oder mit einem beliebigen Druckverfahren, wie z. B. Siebdruck, Flexodruck, Nozzle Printing oder Offsetdruck, besonders bevorzugt aber LITI (Light Induced Thermal Imaging, Thermotransferdruck) oder Ink-Jet Druck (Tintenstrahldruck), hergestellt werden.

Weiterhin bevorzugt ist es, dass zur Herstellung einer erfindungsgemäßen organischen Elektrolumineszenzvorrichtung eine oder mehrere Schichten aus Lösung und eine oder mehrere Schichten durch ein Sublimationsverfahren aufgetragen werden.

Aufgrund der guten Löslichkeit der Verbindungen gemäß Formel (I) ist es bevorzugt, dass die Schicht enthaltend eine oder mehrere Verbindungen der Formel (I) aus Lösung aufgebracht wird. Bevorzugt ist dies die emittierende Schicht einer organischen Elektrolumineszenzvorrichtung.

Erfindungsgemäß können die elektronischen Vorrichtungen enthaltend eine oder mehrere erfindungsgemäße Verbindungen in Displays, als Lichtquellen in Beleuchtungsanwendungen sowie als Lichtquellen in medizinischen und/oder kosmetischen Anwendungen (z.B. Lichttherapie) eingesetzt werden.

### Ausführungsbeispiele

### A) Synthesebeispiele

### A-1) Synthese des Grundgerüsts der Verbindungen

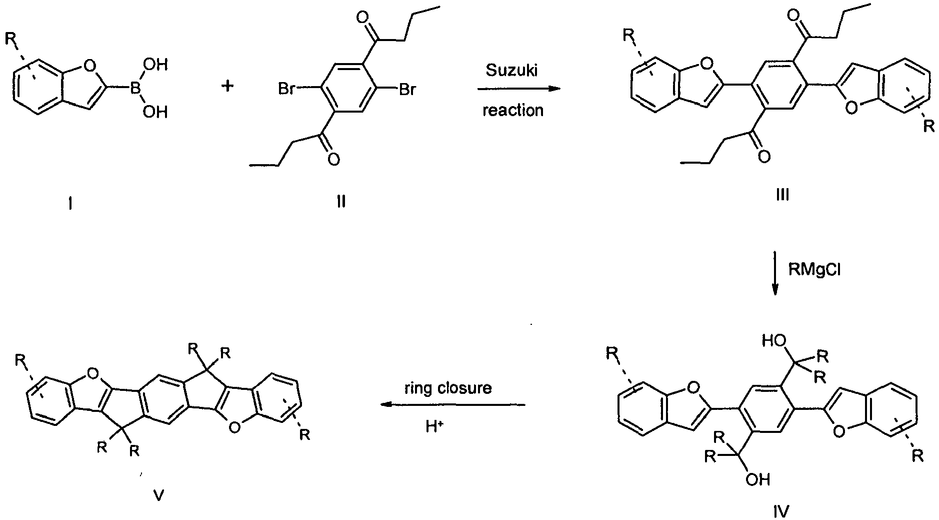

### A-1-1) Synthese der Verbindung I:

Die Verbindung mit R = H (Ia) ist kommerziell erhältlich. Analoge Verbindungen mit R ≠ H sind, sofern sie nicht kommerziell erhältlich sind, nach dem folgenden Verfahren oder nach Verfahren, die in WO 2012/165612 A1, A. S. K. Hashmi, Tetrahedron, 65 (2009) 9021-9029, oder Org. Biomol. Chem. 2014, 12, 4747-4753 beschrieben sind, erhältlich:
Für R ≠ H, z. B. unten gezeigte Verbindung Ib, wird die Synthese wie folgt durchgeführt:

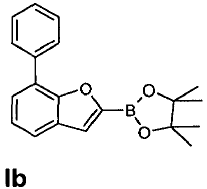

### A-1-1-1) Allgemeines Schema für Synthese von Verbindungen des Typs I

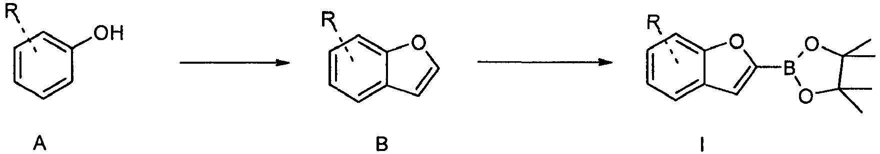

### A-1-1-2) Konkretes Verfahren zur Herstellung der Verbindung Ib

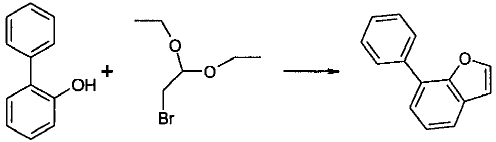

In einem 500 mL Zweihalskoben werden 35 g (206 mmol) Biphenyl-2-ol, 85.3 g (617 mmol) Kaliumcarbonat und 47 mL (308 mmol) 2-Brom-1,1-diethoxy-ethan in 200 mL wasserfreiem DMF vorgelegt und 16 Stunden bis zum vollständigen Umsatz refluxiert. Anschließend wird die Reaktion auf Raumtemperatur abgekühlt und mit 400 mL Wasser versetzt. Die organische Phase wird mit 300 mL Toluol erweitert. Die Phasen werden getrennt und die wässrige Phase wird zweimal mit 200 mL Toluol extrahiert. Die vereinten organischen Phasen werden dreimal mit 20%iger Natriumhydroxidlösung gewaschen, über Kieselgel filtriert und unter vermindertem Druck bis zur Trockene eingeengt. Der Rückstand wird in 600 mL wasserfreiem Toluol gelöst und in einem 1 L Zweihalskolben mit Wasserabscheider vorgelegt, mit 5 g Amberlyst 15 versetzt und 15 Stunden bei 125 °C gerührt. Das Reaktionsgemisch wird nach Abkühlen auf Raumtemperatur filtriert und unter vermindertem Druck eingeengt. Nach säulenchromatographischer Aufreinigung (Heptan/Toluol 9:1) werden 31 g (160 mmol; 78 % d. Th.) farbloses Öl erhalten.

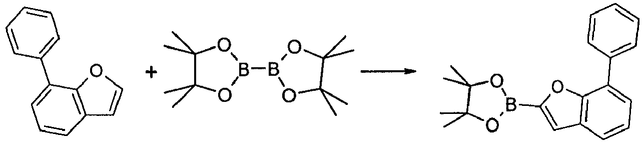

In einem 1 L Zweihalskolben werden 36 g (185 mmol) der oben erhaltenen Verbindung und 24.7 g (97 mmol) Bis-pinacolato-diboron in 400 mL Heptan vorgelegt. Anschließend werden 123 mg (0.19 mmol) (1,5-Cyclooctadiene)(methoxy)iridium(I) dimer und 99.5 mg (037 mmol) 4,4'-Di-tert-butyl-2,2'-dipyridyl zugegeben und über 16 h bei 35 °C gerührt. Der ausgefallene Feststoff wird abgesaugt und mit Heptan gewaschen und unter vermindertem Druck getrocknet. Ausbeute: 51 g (159 mmol; 86 % d. Th.) farbloser Feststoff.

### A-1-2) Synthese von III am Beispiel von 2,5-Bis-benzofuran-2-ylterephthalsäurediethylester (Ar=H) (IIIa)

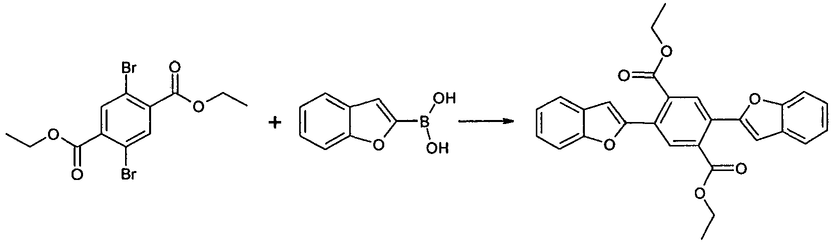

In einem 2 L Vierhalskolben werden 35 g (0.09 mol) 2,5-Dibromterephthalsäurediethylester, 33.5 g (0.2 mmol) Benzofuran-2-ylboronsäure und 46.9 g (0.19 mol) Trikaliumphosphat Monohydrat in 800 mL Toluol/Dioxan/Wasser (2:1:1) vorgelegt. Nach Zugabe von 413 mg (1.8 mmol) Palladiumacetat und 3.36 g (11 mmol) wird eine Stunde refluxiert. Anschließend wird die Reaktion auf Raumtemperatur abgekühlt und die organische Phase mit Ethylacetat erweitert. Die Phasen werden getrennt und die wässrige Phase wird zweimal mit 300 mL Ethylacetat extrahiert. Die vereinten organischen Phasen werden mit Wasser gewaschen, über Natriumsulfat getrocknet, filtriert und unter vermindertem Druck eingeengt. Der Rückstand wird mit 400 mL Methanol versetzt und 20 Minuten bei 60 °C gerührt. Nach Abkühlen auf Raumtemperatur wird der Feststoff filtriert und unter vermindertem Druck getrocknet. Ausbeute: 40.9 g (0.09 mol, 98 %) gelber Feststoff.

Analog können auch folgende Verbindungen hergestellt werden:

| | Edukt 1 | Edukt 2 | Produkt | Ausbeute |
|---|---|---|---|---|
| **IIIa** | 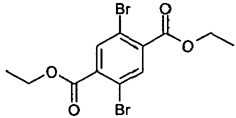 | 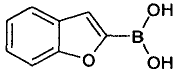 | 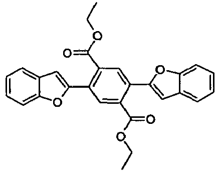 | 98 % |
| | CAS 18013-97-3 | | | |
| **IIIb** | 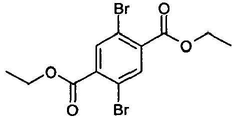 | 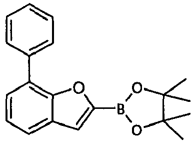 | 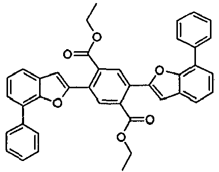 | 91 % |

### A-1-3) Synthese von IV am Beispiel von 2-[2,5-Bis-benzofuran-2-yl-4-(1-hydroxy-1-methyl-ethyl)-phenyl]-propan-2-ol (Ar= H) (IVa)

In einem 4 L Vierhalskolben werden 46.6 g (0.2 mol) wasserfreies Ceriumchlorid in 300 mL wasserfreiem THF vorgelegt und auf 0 °C gekühlt. Anschließend werden 40.9 g (0.09 mol) 2,5-Bis-benzofuran-2-ylterephthalsäurediethylester, gelöst in 700 mL wasserfreiem THF, langsam hinzu getropft, sodass die Innentemperatur unter 5 °C bleibt. Nach vollständiger Zugabe wird eine Stunde bei dieser Temperatur gerührt. Nach vollständigem Umsatz werden 400 mL gesättigte Ammoniumchloridlösung hinzu getropft, sodass die Temperatur unter 20°C bleibt. Die erhaltene Suspension wird filtriert und der Feststoff mit Ethylacetat gewaschen. Das Filtrat wird gründlich mit Ethylacetat gewaschen und anschließend die Phasen getrennt. Die wässrige Phase wird zweimal mit Ethylacetat (200 mL) extrahiert. Die vereinten organischen Phasen werden über Natriumsulfat getrocknet und anschließend unter vermindertem Druck eingeengt. Nach Zugabe von 400 mL Heptan wird anschließend bei 60 °C für 30 Minuten gerührt. Das gewünschte Produkt fällt als hellgelber Feststoff aus. Ausbeute: 35 g (82 mmol, 92 %)

Analog können auch folgende Verbindungen hergestellt werden:

| | Edukt 1 | Edukt 2 | Produkt | Ausbeute |
|---|---|---|---|---|
| **IVa** | 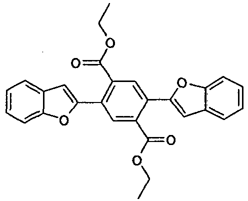 | MeMgCl | 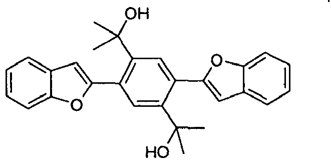 | 92 % |
| **IVb** | 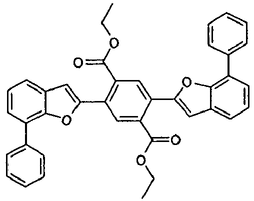 | MeMgCl | 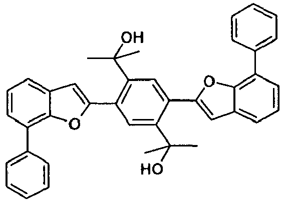 | 79 % |

### A-1-4) Synthese von V am Beispiel von Ar=H (Va)

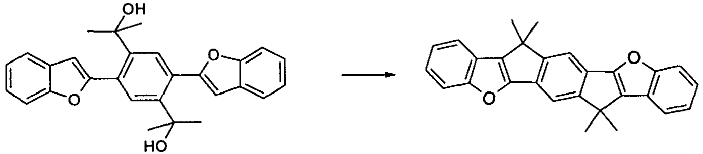

In einem 2 L Vierhalskolben werden 24 g (0.25 mol) Polyphosphorsäure in 200 mL Dichlormethan vorgelegt und auf 0 °C gekühlt. Bei dieser Temperatur werden 16 mL Methansulfonsäure zugetropft. Anschließend werden 35 g (0.08 mol) **IVa**, gelöst in 900 mL Dichlormethan, langsam bei 0 °C zugetropft und zwei Stunden bei dieser Temperatur weitergerührt. Nach vollständigem Umsatz wird die Reaktion mit 100 mL Ethanol versetzt und 30 Minuten gerührt. Das Reaktionsgemisch werden weitere 700 mL Ethanol zugefügt und das Dichlormethan wird unter vermindertem Druck entfernt. Der ausgefallene Feststoff wird filtriert. Die weitere Aufreinigung erfolgt mittels Heißextraktion über Aluminiumoxid mit Toluol/Heptan 1:2 und mehrfacher Umkristallisation aus Heptan/Toluol. Nach zweimaliger Sublimation bei 10⁻⁵ bar und >250 °C wird das Produkt in einer HPLC-Reinheit >99.9 % erhalten. Ausbeute: 8.4 g (22 mmol; 27 %) hellgelber Feststoff

Analog können folgende Verbindungen hergestellt werden:

| | Edukt 1 | Produkt | Ausbeute |
|---|---|---|---|
| **Va** | 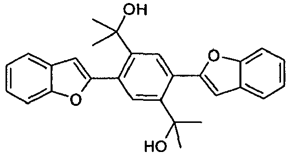 | 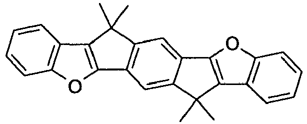 | 27 % |
| **Vb** | 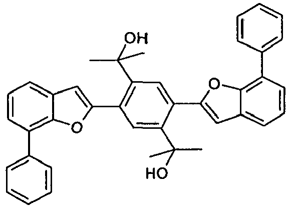 | 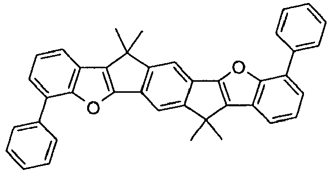 | 52 % |

### A-2) Einführung von Substituenten am Grundgerüst der Verbindung durch Bromierung und Suzuki-Reaktion

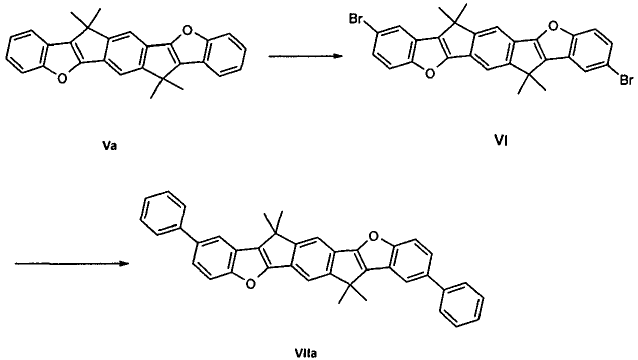

### A-2-1) Bromierung von Va am Beispiel von VI:

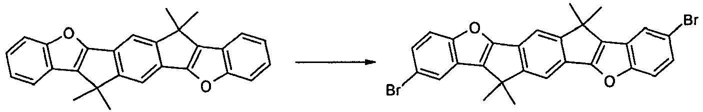

In einem 1 L Vierhalskolben werden 15 g (38.5 mmol) **Va** in 400 mL Dichlormethan vorgelegt und mit 14 g (78.6 mmol) NBS und 568 mg (1.9 mmol) Eisen(III)bromid versetzt und 18 Stunden bei Raumtemperatur gerührt. Das Reaktionsgemisch wird filtriert und der Feststoff mit Ethanol gewaschen. Das Filtrat wird unter vermindertem Druck eingeengt und der ausgefallene Feststoff abfiltriert und mit etwas Ethanol gewaschen. Beide Feststoffe werden vereint, in Toluol gelöst und über Aluminiumoxid filtriert. Nach weiterer Umkristallisation aus Heptan und Toluol wird das Produkt als gelber Feststoff erhalten.
Ausbeute: 11.7 g (21 mmol; 56 %)

### A-2-2) Synthese von VIIa:

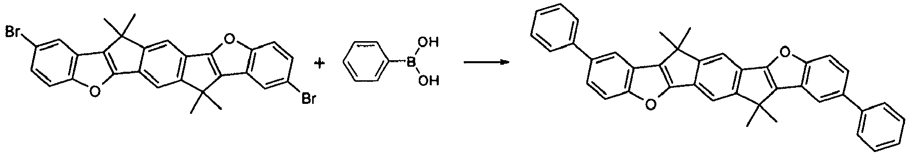

In einem 1 L Vierhalskolben werden 20 g (36 mmol) **VIa,** 10 g (82 mmol) Phenylboronsäure und 18.6 g (81 mmol) Trikaliumphosphat Monohydrat in 600 mL Toluol/Dioxan/Wasser (1:1:1) vorgelegt. Anschließend werden 161 mg (0.72 mmol) Palladiumacetat und 1.3 g (4.32 mmol) Tri-o-tolylphosphin zugegeben und 16 h bis zum vollständigen Umsatz refluxiert. Nach Abkühlen auf Raumtemperatur wird die organische Phase mit 300 mL Ethylacetat erweitert und die Phasen getrennt. Die wässrige Phase wird zweimal mit Ethylacetat extrahiert. Die vereinten organischen Phasen werden über Natriumsulfat getrocknet und anschließend unter vermindertem Druck eingeengt. Anschließend wird der erhaltene Feststoff über Aluminiumoxid heiß extrahiert (Toluol/Heptan). Der ausgefallene Feststoff wird filtriert und mehrmals aus Toluol/Heptan umkristallisiert. Nach zweimaliger Sublimation (10⁻⁶ bar >300 °C) wird das Produkt als hellgelber Feststoff mit einer HPLC-Reinheit >99.9 % erhalten. Ausbeute: 9.3 g (17 mmol; 48 %).

Analog können folgende Verbindungen hergestellt werden:

| | Edukt 1 | Edukt 2 | Produkt VII | Ausbeute |
|---|---|---|---|---|
| **VIIa** | 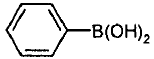 | **VI** | 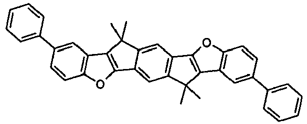 | 48 % |
| **VIIb** | 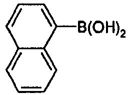 | **VI** | 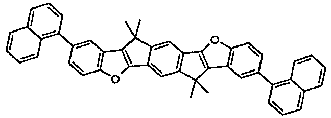 | 37 % |
| **VIIc** | 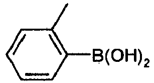 | **VI** | 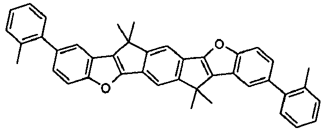 | 46 % |
| **VIId** | 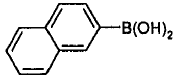 | **VI** | 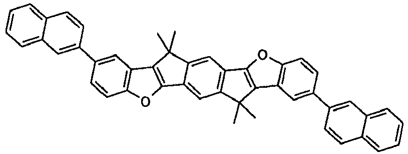 | 43 % |
| **VIIe** | 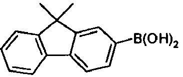 | **VI** | 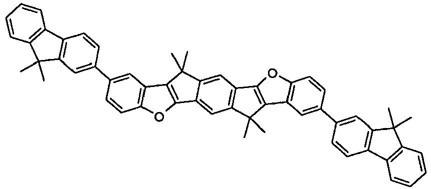 | 49 % |
| **VIIf** | 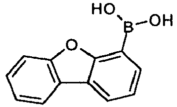 | **VI** | 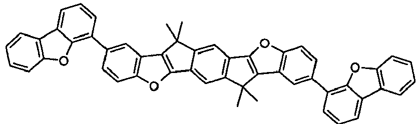 | 47 % |

### B) Device-Beispiele

Die Herstellung von erfindungsgemäßen OLEDs sowie OLEDs nach dem Stand der Technik erfolgt nach einem allgemeinen Verfahren gemäß WO 04/058911, das auf die hier beschriebenen Gegebenheiten (Schichtdickenvariation, Materialien) angepasst wird.

In den folgenden Beispielen (siehe Tabellen 1 bis 3) werden die Daten verschiedener OLEDs vorgestellt. Als Substrate werden Glassubstrate verwendet, die mit strukturiertem ITO (Indium Zinn Oxid) der Dicke 50 nm beschichtet sind. Die OLEDs haben prinzipiell folgenden Schichtaufbau: Substrat / Buffer / Lochinjektionsschicht 1 (95% HTL1+ 5% HIL, 20 nm) / Lochtransportschicht (HTL2, Dicke in Tabelle 1 angegeben) / Emissionsschicht (EML, 20 nm) / Elektronentransportschicht (ETL, 20 nm) / Elektroneninjektionsschicht (EIL, 3 nm) und abschließend eine Kathode. Die Kathode wird durch eine 100 nm dicke Aluminiumschicht gebildet. Als Buffer wird eine 20 nm dicke Schicht Clevios P VP AI 4083 (bezogen von Heraeus Clevios GmbH, Leverkusen) durch Spincoating aufgebracht. Alle restlichen Materialien werden in einer Vakuumkammer thermisch aufgedampft. Der Aufbau der OLEDs ist in Tabelle 1 gezeigt. Die verwendeten Materialien sind in Tabelle 3 gezeigt.

Die Emissionsschicht (EML) besteht immer aus mindestens einem Matrixmaterial (Host=H) und einer emittierenden Verbindung (Dotand=D), die dem Matrixmaterial durch Coverdampfung in einem bestimmten Volumenanteil beigemischt wird. Eine Angabe wie H1:D1 (95%:5%) bedeutet hierbei, dass das Material H1 in einem Volumenanteil von 95% und D1 in einem Anteil von 5% in der Schicht vorliegt.

Die OLEDs werden standardmäßig charakterisiert. Hierfür werden die Elektrolumineszenzspektren aufgenommen, die Stromeffizienz (gemessen in cd/A) und die externe Quanteneffizienz (EQE, gemessen in Prozent) in Abhängigkeit der Leuchtdichte unter Annahme einer lambertschen Abstrahlcharakteristik aus Strom-Spannungs-Leuchtdichte-Kennlinien (IUL-Kennlinien) berechnet. Die Elektrolumineszenzspektren werden bei einer Leuchtdichte von 1000 cd/m² aufgenommen und daraus die CIE 1931 x und y Farbkoordinaten berechnet. Die Angabe EQE @ 1000 cd/m² bezeichnet die externe Quanteneffizienz bei einer Betriebsleuchtdichte von 1000 cd/m². Die erhaltenen Daten für die verschiedenen OLEDs sind in Tabelle 2 zusammengefasst.

### Verwendung der erfindungsgemäßen Verbindungen als Matrix in fluoreszierenden OLEDs

Es werden die erfindungsgemäßen Verbindungen H1 und H2 einzeln als Matrix in der emittierenden Schicht von OLEDs eingesetzt (Struktur s. Tabelle 3). Als Emittermaterial der emittierenden Schicht wird dabei die Verbindung D verwendet. Die erhaltenen OLEDs sind E1 und E2. Sie zeigen sehr gute externe Quanteneffizienten (EQE) bei tiefblauer Emission (Tabelle 2).

### Verwendung der erfindungsgemäßen Verbindungen als Lochtransportmaterialien in OLEDs

Beispiel E3, in dem die erfindungsgemäße Verbindung H1 als Lochtransportmaterial in der Lochtransportschicht eingesetzt wird, zeigt ebenfalls eine gute externe Quanteneffizienz bei tiefblauer Emission (Tabelle 2). Dies belegt die gute Eignung der erfindungsgemäßen Verbindungen als lochtransportierende Verbindungen.

| **Tabelle 1: Aufbau der OLEDs** | | |
|---|---|---|
| ***Bsp.*** | ***HTL (20 nm)*** | ***EML (Dicke* / *20nm)*** |
| E1 | HTL2 | H-1(95%):D(5%) |
| E2 | HTL2 | H-2(95%):D(5%) |
| E3 | H-1 | BH (95%):D(5%) |

| **Tabelle 2: Daten der OLEDs** | | | |
|---|---|---|---|
| ***Bsp.*** | ***EQE [%]* @ *1000 cd*/*m²*** | ***CIE x*** | ***CIE y*** |
| E1 | 6.3 | 0.139 | 0.143 |
| E2 | 6.5 | 0.142 | 0.135 |
| E3 | 5.7 | 0.142 | 0.158 |

| **Tabelle 3: Strukturen der verwendeten Materialien** | |
|---|---|
| 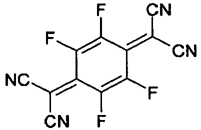 | 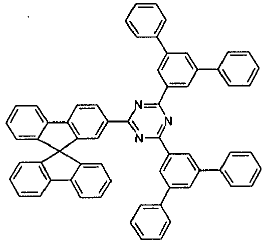 |
| HIL | ETL |
| 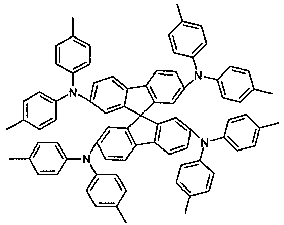 | 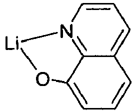 |
| HTL1 | EIL |
| 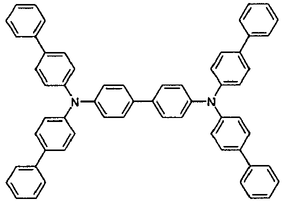 | |
| HTL2 | |
| 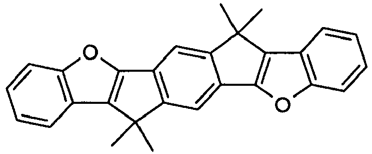 | 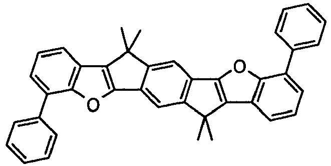 |
| H-1 | H-2 |
| 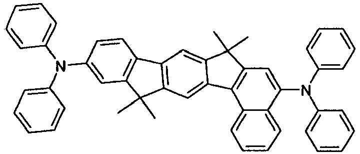 | 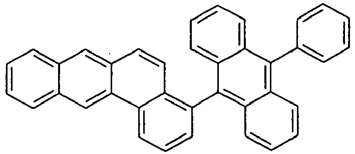 |
| D | BH |

## Patentansprüche

1. Verbindung gemäß einer Formel (I) 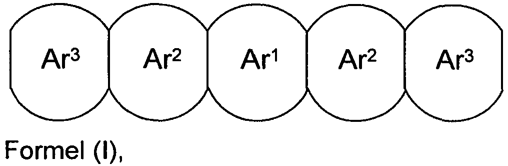 wobei für die auftretenden Variablen gilt: 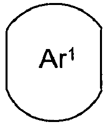 ist ein Benzolring, der jeweils mit Resten R¹ substituiert sein kann; 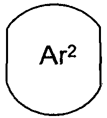 ist bei jedem Auftreten gleich oder verschieden gewählt aus Einheiten der Formel (Ar²-1) oder (Ar²-2) 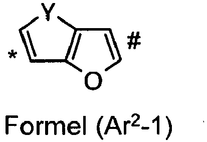 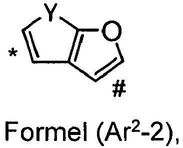 wobei die mit * markierte Bindung jeweils diejenige Bindung ist, über die die Einheit an die Gruppe Ar¹ ankondensiert ist, und die mit # markierte Bindung jeweils diejenige Bindung ist, über die die Einheit an die Gruppe Ar³ ankondensiert ist;
Y ist bei jedem Auftreten gleich oder verschieden C(R²)₂ oder Si(R²)₂; 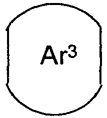 ist bei jedem Auftreten gleich oder verschieden gewählt aus aromatischen Ringsystemen mit 6 bis 30 aromatischen Ringatomen oder heteroaromatischen Ringsystemen mit 5 bis 30 aromatischen Ringatomen, die jeweils mit Resten R³ substituiert sein können;
R¹, R², R³ sind bei jedem Auftreten gleich oder verschieden gewählt aus H, D, F, C(=O)R⁴, CN, Si(R⁴)₃, N(R⁴)₂, P(=O)(R⁴)₂, OR⁴, S(=O)R⁴, S(=O)₂R⁴, geradkettigen Alkyl- oder Alkoxygruppen mit 1 bis 20 C-Atomen, verzweigten oder cyclischen Alkyl- oder Alkoxygruppen mit 3 bis 20 C-Atomen, Alkenyl- oder Alkinylgruppen mit 2 bis 20 C-Atomen, aromatischen Ringsystemen mit 6 bis 40 aromatischen Ringatomen, und heteroaromatischen Ringsystemen mit 5 bis 40 aromatischen Ringatomen; wobei zwei oder mehr Reste R¹, R² bzw. R³ miteinander verknüpft sein können und einen Ring bilden können; wobei die genannten Alkyl-, Alkoxy-, Alkenyl- und Alkinylgruppen und die genannten aromatischen Ringsysteme und heteroaromatischen Ringsysteme jeweils mit einem oder mehreren Resten R⁴ substituiert sein können; und wobei eine oder mehrere CH₂-Gruppen in den genannten Alkyl-, Alkoxy-, Alkenyl- und Alkinylgruppen durch -R⁴C=CR⁴-, -C≡C-, Si(R⁴)₂, C=O, C=NR⁴, -C(=O)O-, -C(=O)NR⁴-, NR⁴, P(=O)(R⁴), -O-, -S-, SO oder SO₂ ersetzt sein können;
R⁴ ist bei jedem Auftreten gleich oder verschieden gewählt aus H, D, F, C(=O)R⁵, CN, Si(R⁵)₃, N(R⁵)₂, P(=O)(R⁵)₂, OR⁵, S(=O)R⁵, S(=O)₂R⁵, geradkettigen Alkyl- oder Alkoxygruppen mit 1 bis 20 C-Atomen, verzweigten oder cyclischen Alkyl- oder Alkoxygruppen mit 3 bis 20 C-Atomen, Alkenyl- oder Alkinylgruppen mit 2 bis 20 C-Atomen, aromatischen Ringsystemen mit 6 bis 40 aromatischen Ringatomen, und heteroaromatischen Ringsystemen mit 5 bis 40 aromatischen Ringatomen; wobei zwei oder mehr Reste R⁴ miteinander verknüpft sein können und einen Ring bilden können; wobei die genannten Alkyl-, Alkoxy-, Alkenyl- und Alkinylgruppen und die genannten aromatischen Ringsysteme und heteroaromatischen Ringsysteme jeweils mit einem oder mehreren Resten R⁵ substituiert sein können; und wobei eine oder mehrere CH₂-Gruppen in den genannten Alkyl-, Alkoxy-, Alkenyl- und Alkinylgruppen durch -R⁵C=CR⁵-, -C≡C-, Si(R⁵)₂, C=O, C=NR⁵, -C(=O)O-, -C(=O)NR⁵-, NR⁵, P(=O)(R⁵), -O-, -S-, SO oder SO₂ ersetzt sein können;
R⁵ ist bei jedem Auftreten gleich oder verschieden gewählt aus H, D, F, CN, Alkylgruppen mit 1 bis 20 C-Atomen, aromatischen Ringsystemen mit 6 bis 40 aromatischen Ringatomen und heteroaromatischen Ringsystemen mit 5 bis 40 aromatischen Ringatomen; wobei zwei oder mehr Reste R⁵ miteinander verknüpft sein können und einen Ring bilden können; und wobei die genannten Alkylgruppen, aromatischen Ringsysteme und heteroaromatischen Ringsysteme mit F oder CN substituiert sein können.

2. Verbindung nach Anspruch 1, **dadurch gekennzeichnet, dass** sie einer Formel (I-C) 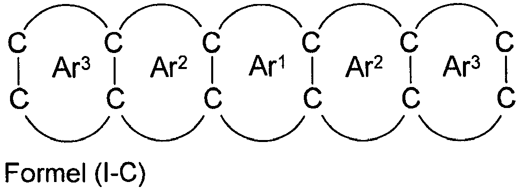 entspricht, wobei die auftretenden Variablen definiert sind wie in Anspruch 1.

3. Verbindung nach Anspruch 1 oder 2, **dadurch gekennzeichnet, dass** sie bezogen auf eine Spiegelebene durch die Mitte und senkrechtstehend auf der Längsachse des langgestreckten Moleküls symmetrisch ist.

4. Verbindung nach einem oder mehreren der Ansprüche 1 bis 3, **dadurch gekennzeichnet, dass** Ar¹ einer der Formeln (Ar¹-1) und (Ar¹-2) entspricht 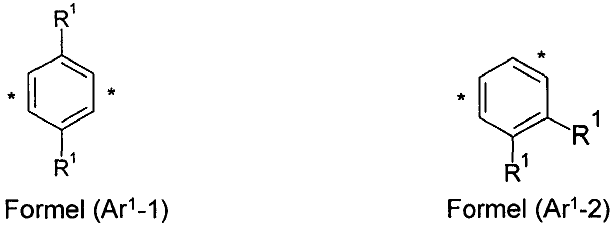 wobei die mit * markierten Bindungen diejenigen Bindungen sind, über die die betreffende Gruppe Ar¹ an die beiden benachbarten Gruppen Ar² ankondensiert ist, und wobei R¹ definiert ist wie in Anspruch 1.

5. Verbindung nach einem oder mehreren der Ansprüche 1 bis 4, **dadurch gekennzeichnet, dass** Ar² gewählt ist aus Gruppen der Formel (Ar²-1), wie gezeigt in Anspruch 1.

6. Verbindung nach einem oder mehreren der Ansprüche 1 bis 5, **dadurch gekennzeichnet, dass** die Gruppe Y gleich C(R²)₂ ist.

7. Verbindung nach einem oder mehreren der Ansprüche 1 bis 6, **dadurch gekennzeichnet, dass** die Gruppen Ar³ bei jedem Auftreten gleich oder verschieden gewählt sind aus den folgenden Gruppen: 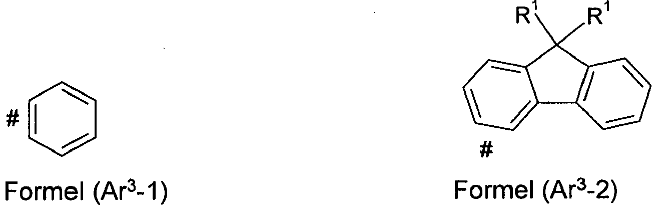 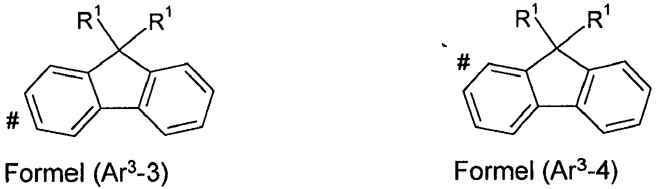 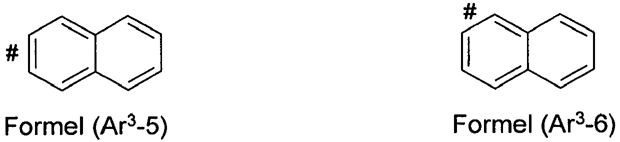 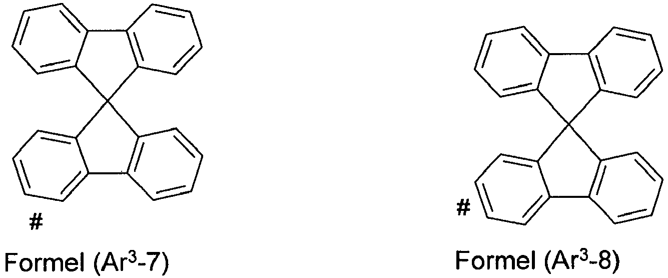 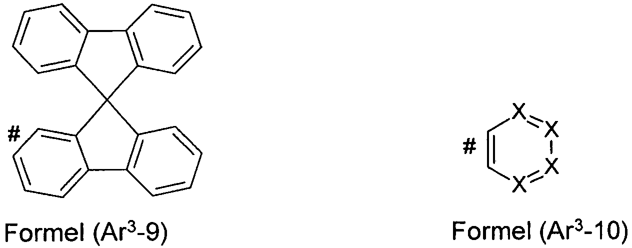 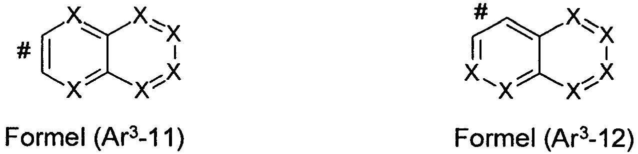 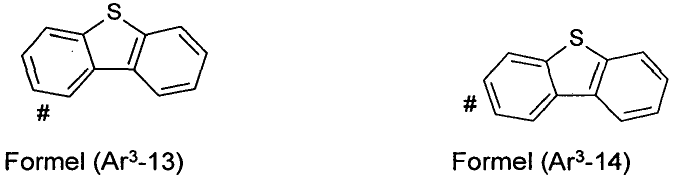 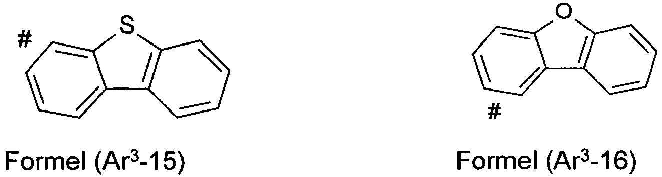 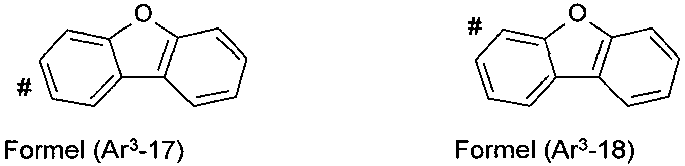 wobei die mit # markierte Bindung diejenige Bindung ist, über die die betreffende Gruppe Ar¹ an die benachbarte Gruppe Ar² ankondensiert ist, wobei die Gruppen an den unsubstituiert gezeigten Positionen mit Resten R³ substituiert sein können, und wobei X bei jedem Auftreten gleich oder verschieden N oder CR³ ist.

8. Verbindung nach einem oder mehreren der Ansprüche 1 bis 7, **dadurch gekennzeichnet, dass** sie der Formel (I-1) 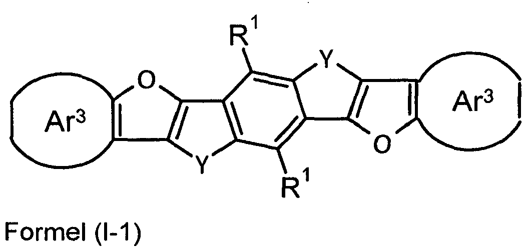 entspricht, wobei die auftretenden Symbole definiert sind wie in Anspruch 1.

9. Verbindung nach einem oder mehreren der Ansprüche 1 bis 8, **dadurch gekennzeichnet, dass** der Wert für das Tripletniveau der Verbindung größer ist als der durch 2 geteilte Wert für das Singulett-Niveau der Verbindung.

10. Verfahren zur Herstellung einer Verbindung nach einem oder mehreren der Ansprüche 1 bis 9, **dadurch gekennzeichnet, dass** die Verbindung durch die folgenden, in der vorliegenden Reihenfolge durchgeführten Schritte gebildet wird:
i) eine metallorganische Kupplungsreaktion zwischen zwei Verbindungen, die je eine Furangruppe enthalten, und einer Verbindung enthaltend die Einheit Ar¹,
ii) die anschließende Reduktion einer in der Verbindung vorhandenen, an die Einheit Ar¹ gebundenen Estergruppe zu einer an ebendiese Einheit Ar¹ gebundenen tertiären Alkoholgruppe, und schließlich
iii) eine Ringschlussreaktion dieser tertiären Alkoholgruppe unter Bildung einer Alkylenbrücke zwischen der Einheit Ar¹ und dem Furanring.

11. Oligomer, Polymer oder Dendrimer enthaltend eine oder mehrere Verbindungen nach einem oder mehreren der Ansprüche 1 bis 9, wobei die Bindung(en) zum Polymer, Oligomer oder Dendrimer an beliebigen, in Formel (I) mit R¹, R² oder R³ substituierten Positionen lokalisiert sein können.

12. Formulierung, enthaltend mindestens eine Verbindung nach einem oder mehreren der Ansprüche 1 bis 9 oder mindestens ein Oligomer, Polymer oder Dendrimer nach Anspruch 11, sowie mindestens ein Lösungsmittel.

13. Elektronische Vorrichtung, ausgewählt aus der Gruppe bestehend aus organischen integrierten Schaltungen (OICs), organischen Feld-Effekt-Transistoren (OFETs), organischen Dünnfilmtransistoren (OTFTs), organischen lichtemittierenden Transistoren (OLETs), organischen Solarzellen (OSCs), organischen optischen Detektoren, organischen Photorezeptoren, organischen Feld-Quench-Devices (OFQDs), organischen lichtemittierenden elektrochemischen Zellen (OLECs), organischen Laserdioden (O-Laser) und organischen Elektrolumineszenzvorrichtungen (OLEDs), enthaltend mindestens eine Verbindung nach einem oder mehreren der Ansprüche 1 bis 9 oder mindestens ein Oligomer, Polymer oder Dendrimer nach Anspruch 11.

14. Elektronische Vorrichtung nach Anspruch 13, ausgewählt aus organischen Elektrolumineszenzvorrichtungen, enthaltend Anode, Kathode, emittierende Schicht und optional weitere organische Schichten, **dadurch gekennzeichnet, dass** die mindestens eine Verbindung oder das mindestens eine Oligomer, Polymer oder Dendrimer als Matrixverbindung in Kombination mit einer oder mehreren emittierenden Verbindungen in der emittierenden Schicht vorliegt, oder als emittierende Verbindung in Kombination mit einer oder mehreren Matrixverbindungen in der emittierenden Schicht vorliegt, oder als lochtransportierende Verbindung in einer zwischen Anode und emittierender Schicht angeordneten Schicht vorliegt.

15. Verwendung einer Verbindung nach einem oder mehreren der Ansprüche 1 bis 9 oder eines Oligomers, Polymers oder Dendrimers nach Anspruch 11 in einer elektronischen Vorrichtung.

## Claims

1. Compound of a formula (I) 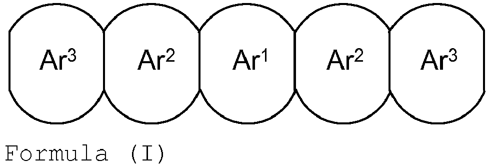 where the variables that occur are: 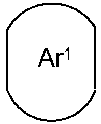 is a benzene ring which may be substituted in each case by R¹ radicals; 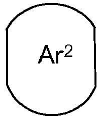 is the same or different at each instance and is selected from units of the formula (Ar²-1) or (Ar²-2) 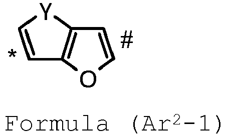 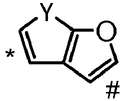 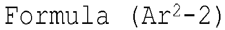
where the bond marked with * is that bond via which the unit is fused to the Ar¹ group, and the bond marked with # is that bond via which the unit is fused to the Ar³ group;
Y is the same or different at each instance and is C(R²)₂ or Si(R²)₂; 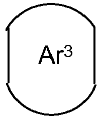 is the same or different at each instance and is selected from aromatic ring systems having 6 to 30 aromatic ring atoms or heteroaromatic ring systems having 5 to 30 aromatic ring atoms, each of which may be substituted by R³ radicals;
R¹, R², R³ are the same or different at each instance and are selected from H, D, F, C(=O)R⁴, CN, Si(R⁴)₃, N(R⁴)₂, P(=O)(R⁴)₂, OR⁴, S(=O)R⁴, S(=O)₂R⁴, straight-chain alkyl or alkoxy groups having 1 to 20 carbon atoms, branched or cyclic alkyl or alkoxy groups having 3 to 20 carbon atoms, alkenyl or alkynyl groups having 2 to 20 carbon atoms, aromatic ring systems having 6 to 40 aromatic ring atoms, and heteroaromatic ring systems having 5 to 40 aromatic ring atoms; where two or more R¹, R² and/or R³ radicals may be joined to one another and may form a ring; where the alkyl, alkoxy, alkenyl and alkynyl groups mentioned and the aromatic ring systems and heteroaromatic ring systems mentioned may each be substituted by one or more R⁴ radicals; and where one or more CH₂ groups in the alkyl, alkoxy, alkenyl and alkynyl groups mentioned may be replaced by -R⁴C=CR⁴-, -C≡C-, Si(R⁴)₂, C=O, C=NR⁴, -C(=O)O-, -C(=O)NR⁴-, NR⁴, P(=O)(R⁴), -O-, -S-, SO or SO₂;
R⁴ is the same or different at each instance and is selected from H, D, F, C(=O)R⁵, CN, Si(R⁵)₃, N(R⁵)₂, P(=O)(R⁵)₂, OR⁵, S(=O)R⁵, S(=O)₂R⁵, straight-chain alkyl or alkoxy groups having 1 to 20 carbon atoms, branched or cyclic alkyl or alkoxy groups having 3 to 20 carbon atoms, alkenyl or alkynyl groups having 2 to 20 carbon atoms, aromatic ring systems having 6 to 40 aromatic ring atoms, and heteroaromatic ring systems having 5 to 40 aromatic ring atoms; where two or more R⁴ radicals may be joined to one another and may form a ring; where the alkyl, alkoxy, alkenyl and alkynyl groups mentioned and the aromatic ring systems and heteroaromatic ring systems mentioned may each be substituted by one or more R⁵ radicals; and where one or more CH₂ groups in the alkyl, alkoxy, alkenyl and alkynyl groups mentioned may be replaced by -R⁵C=CR⁵-, -C≡C-, Si(R⁵)₂, C=O, C=NR⁵, -C(=O)O-, -C(=O)NR⁵⁻, NR⁵, P(=O)(R⁵), -O-, -S-, SO or SO₂;
R⁵ is the same or different at each instance and is selected from H, D, F, CN, alkyl groups having 1 to 20 carbon atoms, aromatic ring systems having 6 to 40 aromatic ring atoms and heteroaromatic ring systems having 5 to 40 aromatic ring atoms; where two or more R⁵ radicals may be joined to one another and may form a ring; and where the alkyl groups, aromatic ring systems and heteroaromatic ring systems mentioned may be substituted by F or CN.

2. Compound according to Claim 1, **characterized in that** it corresponds to a formula (I-C) 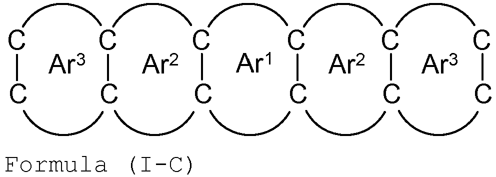 where the variables that occur are as defined in Claim 1.

3. Compound according to Claim 1 or 2, **characterized in that** it is symmetric with respect to a mirror plane through the middle and at right angles to the longitudinal axis of the elongated molecule.

4. Compound according to one or more of Claims 1 to 3, **characterized in that** Ar¹ corresponds to one of the formulae (Ar¹-1) and (Ar¹-2) 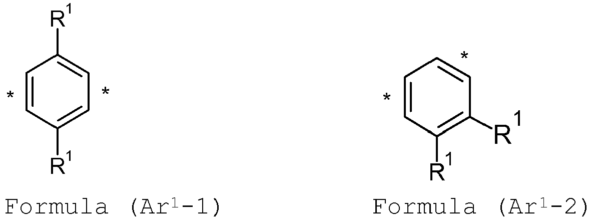 where the bonds marked with * are those bonds via which the Ar¹ group in question is fused to the two adjacent Ar² groups, and where R¹ is as defined in Claim 1.

5. Compound according to one or more of Claims 1 to 4, **characterized in that** Ar² is selected from groups of the formula (Ar²-1) as shown in Claim 1.

6. Compound according to one or more of Claims 1 to 5, **characterized in that** the Y group is C(R²)₂.

7. Compound according to one or more of Claims 1 to 6, **characterized in that** the Ar³ groups are the same or different at each instance and are selected from the following groups: 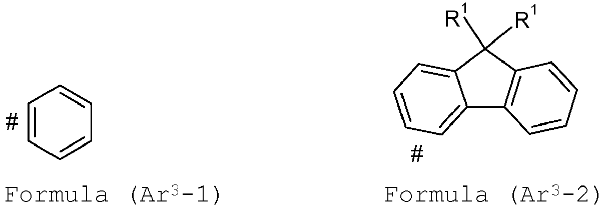 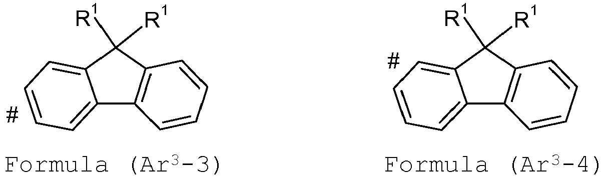 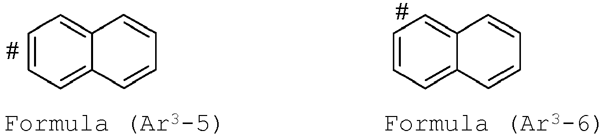 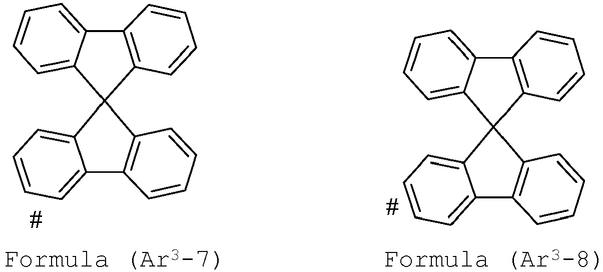 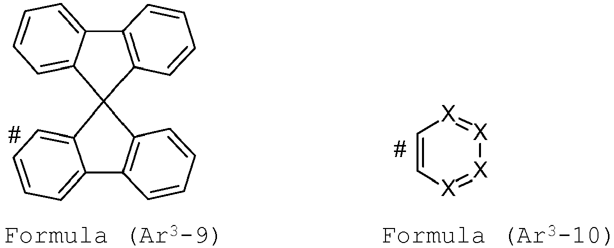 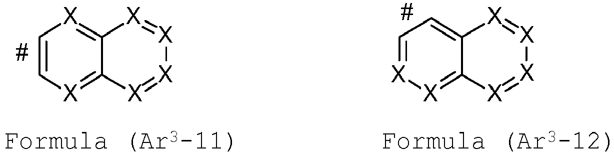 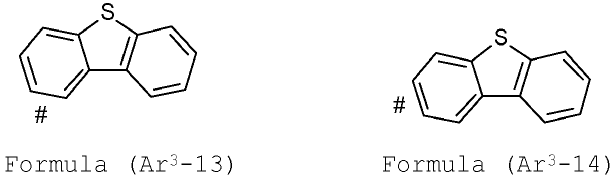 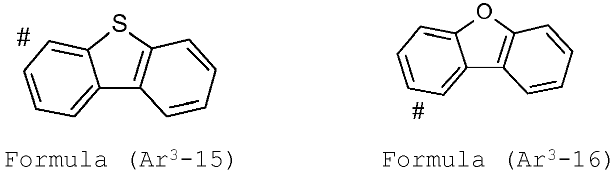 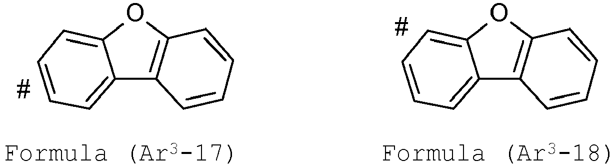 where the bond marked with # is that bond via which the Ar¹ group in question is fused to the adjacent Ar² group, where the groups may be substituted by R³ radicals at the positions shown as unsubstituted, and where X is the same or different at each instance and is N or CR³.

8. Compound according to one or more of Claims 1 to 7, **characterized in that** it corresponds to the formula (I-1) 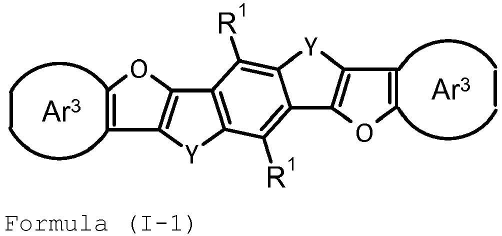 where the symbols that occur are as defined in Claim 1.

9. Compound according to one or more of Claims 1 to 8, **characterized in that** the value of the triplet level of the compound is greater than the value of the singlet level of the compound divided by 2.

10. Process for preparing a compound according to one or more of Claims 1 to 9, **characterized in that** the compound is formed by the following steps conducted in the present sequence:
i) an organometallic coupling reaction between two compounds each containing a furan group and a compound containing the Ar¹ unit,
ii) the subsequent reduction of an ester group bonded to the Ar¹ unit and present in the compound to a tertiary alcohol group bonded to the same Ar¹ unit, and finally
iii) a ring-closure reaction of this tertiary alcohol group to form an alkylene bridge between the Ar¹ unit and the furan ring.

11. Oligomer, polymer or dendrimer containing one or more compounds according to one or more of Claims 1 to 9, wherein the bond(s) to the polymer, oligomer or dendrimer may be localized at any desired positions substituted by R¹, R² or R³ in formula (I) .

12. Formulation comprising at least one compound according to one or more of Claims 1 to 9, or at least one oligomer, polymer or dendrimer according to Claim 11, and at least one solvent.

13. Electronic device selected from the group consisting of organic integrated circuits (OICs), organic field-effect transistors (OFETs), organic thin-film transistors (OTFTs), organic light-emitting transistors (OLETs), organic solar cells (OSCs), organic optical detectors, organic photoreceptors, organic field-quench devices (OFQDs), organic light-emitting electrochemical cells (OLECs), organic laser diodes (O-lasers) and organic electroluminescent devices (OLEDs), comprising at least one compound according to one or more of Claims 1 to 9 or at least one oligomer, polymer or dendrimer according to Claim 11.

14. Electronic device according to Claim 13, selected from organic electroluminescent devices comprising anode, cathode, emitting layer and optionally further organic layers, **characterized in that** the at least one compound or the at least one oligomer, polymer or dendrimer is present as matrix compound in combination with one or more emitting compounds in the emitting layer, or is present as emitting compound in combination with one or more matrix compounds in the emitting layer, or is present as hole-transporting compound in a layer arranged between anode and emitting layer.

15. Use of a compound according to one or more of Claims 1 to 9, or of an oligomer, polymer or dendrimer according to Claim 11, in an electronic device.

## Revendications

1. Composé selon une formule (I) 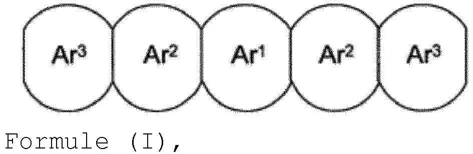 étant entendu que pour les variables représentées : 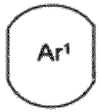 représente un cycle benzénique, pouvant à chaque fois être substitué par des radicaux R¹ ; 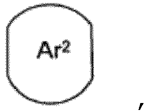 identique ou différent en chaque occurrence, est choisi parmi les unités de la formule (Ar²-1) ou de la formule (Ar²-2) 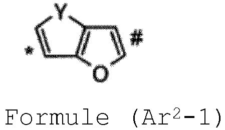 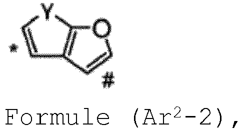
la liaison marquée par * représentant à chaque fois la liaison par laquelle l'unité est condensée au groupe Ar¹ et la liaison marquée par # représentant à chaque fois la liaison par laquelle l'unité est condensée au groupe Ar³ ;
Y, identique ou différent en chaque occurrence, représente C(R²)₂ ou Si(R²)₂ ; 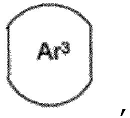 identique ou différent en chaque occurrence, est choisi parmi les systèmes cycliques aromatiques possédant 6 jusqu'à 30 atomes de cycle aromatique ou parmi les systèmes cycliques hétéroaromatiques possédant 5 jusqu'à 30 atomes de cycle aromatique pouvant être à chaque fois substitués par des radicaux R³ ;
R¹, R², R³, identiques ou différents en chaque occurrence, sont choisis parmi H, D, F, C(=O)R⁴, CN, Si(R⁴)₃, N(R⁴)₂, P(=O)(R⁴)₂, OR⁴, S(=O)R⁴, S(=O)₂R⁴, les groupes C₁₋₂₀-alkyle ou C₁₋₂₀-alcoxyle linéaires, les groupes C₃₋₂₀-alkyle ou C₃₋₂₀-alcoxyle ramifiés ou cycliques, les groupes C₂₋₂₀-alcényle ou C₂₋₂₀-alcynyle, les systèmes cycliques aromatiques possédant 6 jusqu'à 40 atomes de cycle aromatique et les systèmes cycliques hétéroaromatiques possédant 5 jusqu'à 40 atomes de cycle aromatique ; deux radicaux R¹, R² ou R³ ou plus pouvant être reliés entre eux et pouvant former un cycle ; lesdits groupes alkyle, alcoxyle, alcényle et alcynyle et lesdits systèmes cycliques aromatiques et lesdits systèmes cycliques hétéroaromatiques pouvant à chaque fois être substitués par un radical ou par plusieurs radicaux R⁴ ; et un ou plusieurs groupe(s) CH₂ dans lesdits groupes alkyle, alcoxyle, alcényle et alcynyle pouvant être remplacé (s) par -R⁴C=CR⁴-, -C≡C-, Si(R⁴)₂, C=O, C=NR⁴, -C(=O)O-, -C(=O)NR⁴-, NR⁴, P(=O)(R⁴), -O-, -S-, SO ou SO₂ ;
R⁴, identique ou différent en chaque occurrence, est choisi parmi H, D, F, C(=O)R⁵, CN, Si(R⁵)₃, N(R⁵)₂, P(=O)(R⁵)₂, OR⁵, S(=O)R⁵, S(=O)₂R⁵, les groupes C₁₋₂₀-alkyle ou C₁₋₂₀-alcoxyle linéaires, les groupes C₃₋₂₀-alkyle ou C₃₋₂₀-alcoxyle ramifiés ou cycliques, les groupes C₂₋₂₀-alcényle ou C₂₋₂₀-alcynyle, les systèmes cycliques aromatiques possédant 6 jusqu'à 40 atomes de cycle aromatique et les systèmes cycliques hétéroaromatiques possédant 5 jusqu'à 40 atomes de cycle aromatique ; deux radicaux R⁴ ou plus pouvant être reliés entre eux et pouvant former un cycle ; lesdits groupes alkyle, alcoxyle, alcényle et alcynyle et lesdits systèmes cycliques aromatiques et lesdits systèmes cycliques hétéroaromatiques pouvant à chaque fois être substitués par un ou plusieurs radical/radicaux R⁵ ; et un ou plusieurs groupe(s) CH₂ dans lesdits groupes alkyle, alcoxyle, alcényle et alcynyle pouvant être remplacé(s) par -R⁵C=CR⁵, -C≡C-, Si(R⁵)₂, C=O, C=NR⁵, -C(=O)O-, -C(=O)NR⁵-, NR⁵, P(=O)(R⁵), -O-, -S-, SO ou SO₂ ;
R⁵, identique ou différent en chaque occurrence, est choisi parmi H, D, F, CN, les groupes C₁₋₂₀-alkyle, les systèmes cycliques aromatiques possédant 6 jusqu'à 40 atomes de cycle aromatique et les systèmes cycliques hétéroaromatiques possédant 5 jusqu'à 40 atomes de cycle aromatique ; deux radicaux R⁵ ou plus pouvant être reliés entre eux et pouvant former un cycle ; lesdits groupes alkyle, lesdits systèmes cycliques aromatiques et lesdits systèmes cycliques hétéroaromatiques pouvant être substitués par F ou CN.

2. Composé selon la revendication 1, **caractérisé en ce qu'**il correspond à une formule (I-C) 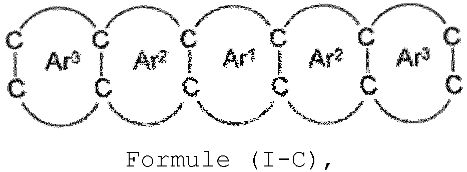 les variables représentées étant définies comme dans la revendication 1.

3. Composé selon la revendication 1 ou 2, **caractérisé en ce qu'**il est symétrique par rapport à un plan-miroir passant par le centre et placé perpendiculairement à l'axe longitudinal de la molécule allongée.

4. Composé selon l'une ou plusieurs des revendications 1 à 3, **caractérisé en ce que** Ar¹ correspond à une des formules (Ar¹-1) et (Ar¹-2) 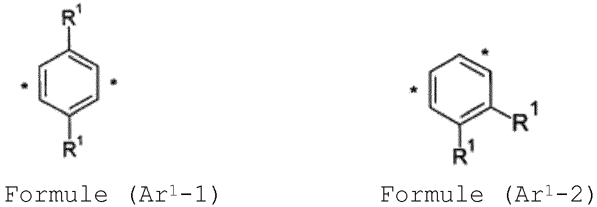 les liaisons marquées par * étant les liaisons par lesquelles les groupes concernés Ar¹ sont condensés aux deux groupes voisins Ar² et R¹ étant tel que défini dans la revendication 1.

5. Composé selon l'une ou plusieurs des revendications 1 à 4, **caractérisé en ce que** Ar² est choisi parmi les groupes de la formule (Ar²-1), tels que représentés dans la revendication 1.

6. Composé selon l'une ou plusieurs des revendications 1 à 5, **caractérisé en ce que** le groupe Y représente C(R²)₂.

7. Composé selon l'une ou plusieurs des revendications 1 à 6, **caractérisé en ce que** les groupes A³, identiques ou différents en chaque occurrence, sont choisis parmi les groupes suivants : 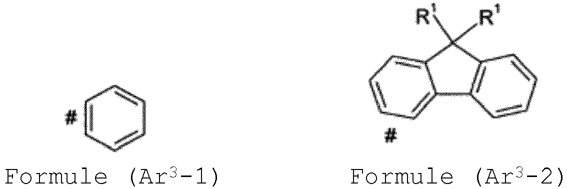 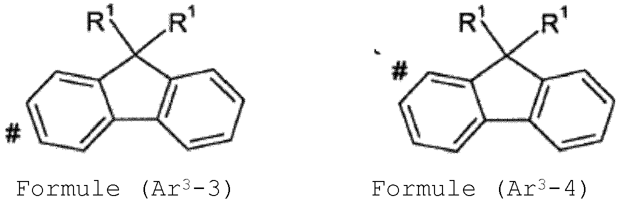 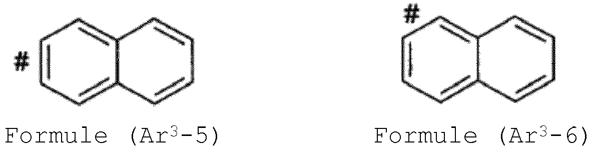 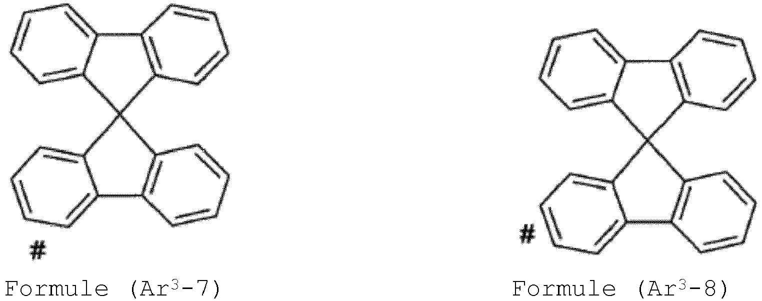 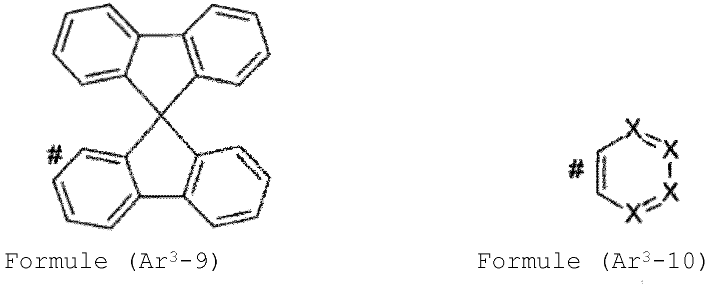 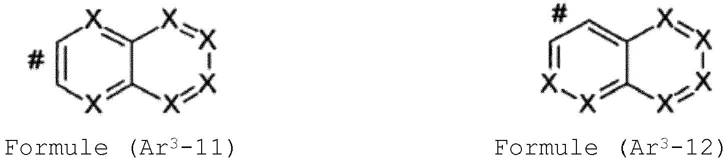 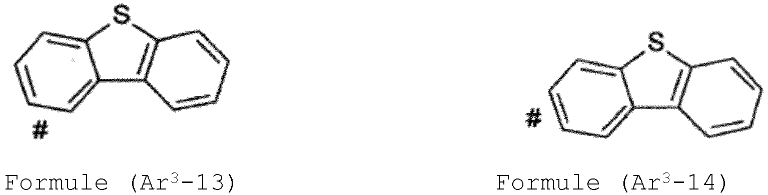 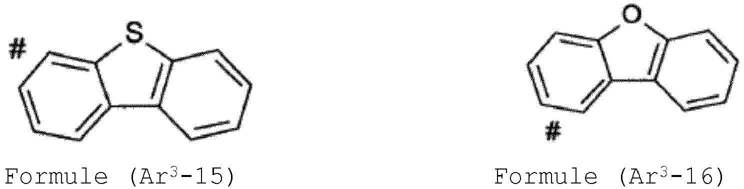 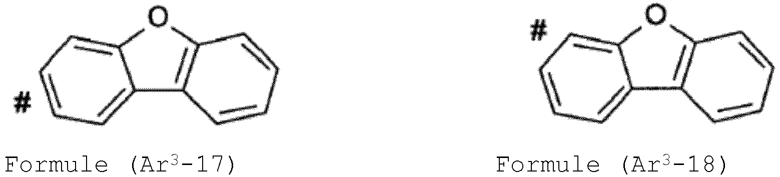 les liaisons marquées par # étant les liaisons par lesquelles les groupes concernés Ar¹ sont condensés aux deux groupes voisins Ar², les groupes pouvant être substitués par des radicaux R³ dans les positions non substituées représentées et X, identique ou différent en chaque occurrence, représentant N ou CR³.

8. Composé selon l'une ou plusieurs des revendications 1 à 7, **caractérisé en ce qu'**il correspond à la formule (I-1) 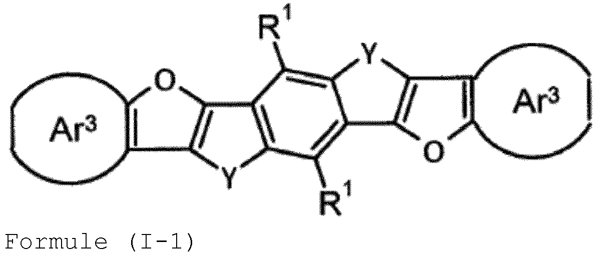 les symboles représentés étant définis comme dans la revendication 1.

9. Composé selon l'une ou plusieurs des revendications 1 à 8, **caractérisé en ce que** la valeur pour le niveau de triplet du composé est supérieure à la valeur divisée par 2 pour le niveau de singulet du composé.

10. Procédé pour la préparation d'un composé selon l'une ou plusieurs des revendications 1 à 9, **caractérisé en ce que** le composé est formé par les étapes suivantes mises en oeuvre selon la présente séquence:
i) une réaction de couplage métallo-organique entre deux composés contenant chacun un groupe furanne et un composé contenant l'unité Ar¹,
ii) la réduction consécutive d'un groupe ester présent dans le composé, lié à l'unité Ar¹ en un groupe alcool tertiaire lié à cette même unité Ar¹ et
enfin
iii) une réaction de fermeture de cycle de ce groupe alcool tertiaire pour former un pont alkylène entre l'unité Ar¹ et le cycle furanne.

11. Oligomère, polymère ou dendrimère contenant un ou plusieurs composés selon l'une ou plusieurs des revendications 1 à 9, la/les liaison(s) au polymère, à l'oligomère ou au dendrimère pouvant être située(s) en l'une quelconque des positions substituées par R¹, R² ou R³ dans la formule (I).

12. Formulation contenant au moins un composé selon l'une ou plusieurs des revendications 1 à 9 ou contenant au moins un oligomère, un polymère ou un dendrimère selon la revendication 11 ainsi qu'au moins un solvant.

13. Dispositif électronique choisi dans le groupe constitué par les circuits intégrés organiques (OIC), les transistors organiques à effet de champ (OFET), les transistors organiques en couches minces (OTFT), les transistors électroluminescents organiques (OLET), les cellules solaires organiques (OSC), les détecteurs optiques organiques, les photorécepteurs organiques, les dispositifs organiques d'extinction de champ (OFQD), les cellules électrochimiques électroluminescentes organiques (OLEC), les diodes laser organiques (O-laser) et les dispositifs électroluminescents organiques (OLED), contenant au moins un composé selon l'une ou plusieurs des revendications 1 à 9 ou contenant au moins un oligomère, un polymère ou un dendrimère selon la revendication 11.

14. Dispositif électronique selon la revendication 13, choisi parmi les dispositifs électroluminescents organiques, contenant une anode, une cathode, une couche émettrice et éventuellement d'autres couches organiques, **caractérisé en ce que** ledit au moins un composé ou ledit au moins un oligomère, polymère ou dendrimère est présent dans la couche émettrice en tant que composé de matrice en combinaison avec un ou plusieurs composé (s) émetteur (s) ou est présent en tant que composé émetteur en combinaison avec un ou plusieurs composé(s) de matrice dans la couche émettrice ou est présent en tant que composés de transport de trous dans une couche disposée entre l'anode et la couche émettrice.

15. Utilisation d'un composé selon l'une ou plusieurs des revendications 1 à 9 ou d'un oligomère, d'un polymère ou d'un dendrimère selon la revendication 11 dans un dispositif électronique.
